(19) **Europäisches Patentamt European Patent Office Office européen des brevets**

(11) **EP 4 621 730 A1**

(12) **DEMANDE DE BREVET EUROPEEN**

(43) Date de publication:
24.09.2025 Bulletin 2025/39

(21) Numéro de dépôt: 24165898.8

(22) Date de dépôt: 25.03.2024

(51) Classification Internationale des Brevets (IPC):
*G06V 10/44* (2022.01) *G06V 10/50* (2022.01)
*G06V 10/82* (2022.01) *G06V 20/69* (2022.01)
*C12Q 1/00* (2006.01)

(52) Classification Coopérative des Brevets (CPC):
**G06V 20/698; C12Q 1/00; G06N 3/045; G06V 10/454; G06V 10/50; G06V 10/82;** G06N 3/0464

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: 22.03.2024 EP 24165656

(71) Demandeurs:
• **bioMérieux**
 **69280 MARCY-L'ETOILE (FR)**
• **Université Jean Monnet Saint-Étienne**
 **42100 Saint-Étienne (FR)**

• **Centre National de la Recherche Scientifique (CNRS)**
 **75016 Paris (FR)**
• **Bioaster**
 **69007 Lyon (FR)**

(72) Inventeurs:
• **SAPAY, Nicolas**
 **69230 Saint-Genis-Laval (FR)**
• **FAURE, Nicolas**
 **38100 Grenoble (FR)**
• **COURBON, Benoit**
 **69007 Lyon (FR)**

(74) Mandataire: **bioMérieux PI Groupement mandataires bioMérieux 69280 Marcy l'Etoile (FR)**

(54) **PROCEDE ET SYSTEME DE CARACTERISATION DE MICROORGANISMES CONTENUS DANS UN ECHANTILLON COMPLEXE**

(57) Un procédé de classification de microorganismes contenus dans un échantillon comprend la préparation d'une lame de l'échantillon, l'acquisition d'au moins une image numérique de la lame et la mise en oeuvre par ordinateur d'un modèle de prédiction de la classe des microorganismes en fonction de l'image acquise.

Selon l'invention, ladite image est subdivisée en sous-images et chaque sous-image est subdivisée en patchs, et:

A. pour chaque patch, l'application d'un extracteur de caractéristiques de microorganismes, formant une partie convolutive d'un premier réseau de neurones convolutif entraîné sur des patchs étant annotés individuellement par au moins une classe;

B. pour chaque sous-image, l'application d'un deuxième réseau de neurones connecté à l'extracteur, comprenant une couche amont de pooling et une ou plusieurs couches aval comprenant une couche de prédiction d'au moins une classe, et entraîné sur des sous-images d'entrainement globalement; et

C. pour l'image acquise:

F.a. le calcul d'un vecteur de caractéristique calculés pour les sous-images;

F.b. l'application d'un modèle de prédiction d'au moins une classe pour les microorganismes.

FIG. 5

EP 4 621 730 A1

**Description**

**DOMAINE DE L'INVENTION**

La présente invention a trait au domaine de l'analyse microbiologique, en particulier de la caractérisation automatique de microorganismes contenus dans un échantillon complexe tel que du sang. Sans être limitatif, l'invention trouve application à la caractérisation du Gram, de la morphologie et de l'agrégation de bactéries, de levures et de champignons infectant un patient ou un animal, notamment des patients suspectés de septicémie.

**ETAT DE LA TECHNIQUE**

Le domaine de l'analyse microbiologique est confronté à de nombreux défis dès lors qu'elle s'écarte des techniques conventionnelles à base de culture sur ou dans un milieu nutritif, par exemple sur boite de Pétri. La détection d'une septicémie déclenchée par une infection bactérienne du sang est un exemple bien connu. La présence de bactéries pathogènes dans le sang peut mener au décès d'un patient, de sorte que leur détection au plus tôt est vital pour ce dernier. Malheureusement, à un stade précoce, la quantité de bactéries est non seulement infime mais en outre ces dernières sont noyées dans une matrice complexe comprenant des globules blancs, rouges, des plaquettes, et bien d'autres éléments de tailles et de quantités significativement plus importantes, rendant à ce jour leur détection difficile, voire impossible. C'est pourquoi une hémoculture, à savoir une étape de pousse des bactéries présentes dans le sang, sang auquel est ajouté un milieu nutritif choisi pour accélérer au plus leur développement, est déclenchée pour tout patient suspecté de septicémie. Cette pousse a pour objectif de multiplier au plus vite les bactéries afin de les rendre détectables. Malheureusement l'hémoculture est un processus encore trop lent au regard de la sévérité de l'infection.

La complexité des échantillons met également en difficulté l'analyse microbiologique dans des applications autrefois routinières dans les laboratoires. C'est le cas notamment de la caractérisation du Gram de bactéries, quel que soit l'échantillon, complexe ou non. En effet, la caractérisation du Gram de bactéries requiert une grande expertise humaine, étant fondée sur l'analyse microscopique de lames de Gram par un technicien qualifié. Or, on observe une perte importante de cette expertise dans un nombre important, voire majoritaire, de laboratoires d'analyse, conduisant à une perte de qualité, voire menant *de facto* à ne plus mettre en oeuvre cette analyse. Pourtant cette dernière, menée très tôt une fois un prélèvement reçu, permet au clinicien de prendre très rapidement des décisions d'antibiothérapies parfois décisives.

La diffusion des techniques d'apprentissage automatisé, et en particulier de l'apprentissage profond à base de réseaux de neurones, est porteuse d'espoir. En effet, ces techniques ont été appliquées avec succès sur des images complexes, comme par exemple la détection de tumeurs pulmonaires. On s'attend ainsi à un progrès analogue dans la microbiologie : la caractérisation de microorganismes présents dans un échantillon à partir d'une image de ce dernier. Pourtant cet espoir se heurte à une barrière de ressources si importante qu'une organisation, si grande soit-elle, éprouve les plus grandes difficultés à mettre au point une solution de caractérisation automatique à base d'intelligence artificielle. En effet, en raison de la taille des bactéries, de leur nombre à un stade précoce et de la complexité de l'échantillon, il est nécessaire d'imager une surface importante avec une très haute résolution. En pratique, on combine pour ce faire plusieurs images brutes de typiquement quelques millions de pixels chacune, et qui ne sont pas nécessairement recouvrantes ni même contiguës, en une seule image composite que nous appellerons simplement « image » par la suite. L'image résultante totalise typiquement plusieurs de dizaines, de millions de pixels à plusieurs milliards de pixels. Quand bien même des techniques de réseaux de neurones convolutifs réduisant la dimensionnalité du problème seraient utilisées, la dimensionalité finale reste si importante que l'apprentissage des architectures classiques nécessiteraient un très grand nombre d'images annotées, sans doute des millions. Outre cette quantité astronomique, l'annotation ne peut être réalisée par des non spécialistes comme c'est le cas par exemple de modèles présents sur Google zoo ou des modèles appris par ubérisation de l'annotation sur des plateformes dédiées. Or, comme évoqué plus haut dans le cas de la caractérisation du Gram, les experts manquent.

A ce jour donc, la mise au point d'outils de caractérisation automatique à base d'intelligence artificielle dans le domaine de l'analyse microbienne, en particulier dans le domaine du *Diagnostic In Vitro* ("IVD"), reste difficile.

**EXPOSE DE L'INVENTION**

Le but de la présente invention est de proposer un procédé de caractérisation automatique de microorganismes présents dans un échantillon se fondant sur l'analyse par intelligence artificielle d'images de très haute résolution, cette analyse étant mise en oeuvre par une architecture d'apprentissage profond entraîné sur un jeu d'apprentissage réduit et permettant un diagnostic performant, en particulier un diagnostic pouvant être qualifié de *Diagnostic In Vitro*

A cet effet, l'invention a pour objet un procédé de classification de microorganismes contenus dans un échantillon, parmi plusieurs classes de microorganismes, le procédé comprenant :

A. la préparation d'une lame, en particulier de microscope, comprenant l'étalement de l'échantillon sur ladite lame ;

B. l'acquisition d'au moins une image numérique de la lame avec une résolution micrométrique ou sub-micrométrique ;

C. l'application, mise en oeuvre par ordinateur, d'un modèle de prédiction de la classe des microorganismes en fonction de l'image acquise.

Selon l'invention, ladite image est subdivisée en une pluralité de sous-images et chaque sous-image est subdivisée en patchs, et l'application du modèle de prédiction comporte :

D. pour chaque patch, l'application d'un extracteur de caractéristiques de microorganismes, ledit extracteur comportant une partie convolutive d'un premier réseau de neurones convolutif, ledit premier réseau étant entraîné sur un ensemble de patchs d'entraînement comprenant des microorganismes, lesdits microorganismes étant annotés individuellement par au moins une classe;

E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval comprenant une couche de prédiction d'au moins une classe, sous la forme d'un score, ledit deuxième réseau étant entraîné sur des sous-images d'entraînement comprenant des microorganismes, chaque sous-image d'entraînement étant globalement annotée par au moins une classe; et

F. pour l'image acquise:

F.a. le calcul d'un vecteur de caractéristique calculé pour les sous-images;

F.b. l'application d'un modèle de prédiction d'au moins une classe pour les microorganismes présents dans l'échantillon en fonction du vecteur de caractéristiques, ledit modèle de prédiction étant entrainé sur des vecteurs de caractéristiques calculés à partir d'images d'entrainement.

En d'autres termes, l'invention propose une architecture d'intelligence artificielle à plusieurs étages spécifiques caractérisés par une annotation de plus en plus faible le long des étages. Cette architecture est entraînée avec seulement quelques centaines de sous-images de haute résolution subdivisées en patchs de dimension réduites (par exemple 256 pixels par 256 pixels ou 224 pixels par 224 pixels), patchs dont une quantité infime (par exemple moins de 5%) sont annotés de manière forte, et ce pour une précision de prédiction globale de plus de 90%.

Selon un mode de réalisation, le vecteur de caractéristiques est une distribution des scores calculés pour les sous-images. Selon un mode de réalisation, chaque image d'entrainement est subdivisée en sous-images et chacune desdites sous-images est subdivisée en patchs, et moins de 50% des patchs des images d'entrainement sont annotés, lesdits patchs annotés formant les patchs d'entrainement du premier réseau convolutif. En particulier, moins de 10% des patchs des images d'entrainement sont annotés. Selon un mode de réalisation, l'entrainement des premier et second réseaux convolutifs et du modèle de prédiction est configuré pour obtenir une spécificité macro de prédiction supérieure ou égale à 90% Selon un mode de réalisation le modèle de prédiction de l'étape F est un modèle « random forest ». Selon un mode de réalisation, la couche amont de pooling est associée à une couche d'attention configurée pour appliquer un poids à la sortie de chaque extracteur. Selon un mode de réalisation, le deuxième réseau est un réseau MII,-CNN entrainé par lots d'instances, les lots d'instances étant constitués des patchs d'entrainement.

Selon un mode de réalisation, le vecteur de caractéristiques de l'étape F.a comporte pour chaque classe :

- le score maximal parmi les sous-images ; et/ou
- le $X^{ième}$ percentile des scores parmi les sous-images, avec X supérieur ou égal à 90%, de préférence égal à 95% ; et/ou
- le $Y^{ème}$ percentile des scores parmi les sous-images, avec Y inférieur ou égal à 10%, de préférence égal à 5% ; et/ou
- le score médian parmi les sous-images.

Selon un mode de réalisation, le vecteur de caractéristiques comprend en outre le nombre de sous-images dans l'image.

Selon un mode de réalisation, le premier réseau convolutif est pré-entraîné sur des images ne comprenant pas de microorganismes puis entraîné sur des patchs d'entrainement annotés. En particulier, le premier réseau convolutif pré-entrainé est un réseau VVG16 ou ResNet.

Selon un mode de réalisation, les microorganismes comprennent des bactéries et les classes comprennent au moins le Gram positif et le Gram négatif, et la préparation de la lame comprend la préparation d'une lame de Gram. En particulier, les classes de microorganismes comprennent en outre des classes de morphotypes. Notamment, les classes de microorganismes comprennent une classe « ni bactérie ni levure », une classe « bacilles à Gram négatif », une classe « bacilles à Gram positif », une classe « bacilles corynée à Gram positif », une classe « coques à Gram négatif », une classe « coques à Gram positif en chaînette », une classe « coques à Gram positif en cluster » et une classe « levures ».

Selon un mode de réalisation, l'échantillon comprend du sang, notamment l'échantillon est une hémoculture positive.

L'invention a également pour objet un procédé d'entrainement d'un modèle de prédiction d'une classe de microorganismes parmi plusieurs classes de microorganismes à partir d'une image numérique d'une lame sur laquelle est étalé un échantillon susceptible de comprendre des microorganismes, ledit procédé d'entraînement comprenant :

A. la constitution d'une base de données d'entrainement comprenant des images numériques de lame annotées par une ou plusieurs classes de microorganismes, les images des lames étant divisées en une pluralité de sous-images annotées par une ou plusieurs classes de microorganismes et les sous-images d'entrainement étant subdivisée en patchs, au moins une partie des patchs étant annotés par une ou plusieurs classes de microorganismes,

B. l'entraînement d'un premier réseau de neurones convolutifs en fonction des patchs annotés ;

C. l'entraînement d'un deuxième réseau de neurones à partir de la base annotée de sous-images, ledit second réseau comprenant au moins un extracteur de caractéristiques de patchs, un étage de pooling des caractéristiques de patchs, et un étage de prédiction de la ou les classes de microorganismes présents sur la sous-image;

D. la constitution d'une base de données de distributions des classes présentes dans les lames d'entrainement en fonction des classes prédites par le deuxième réseau de neurones appliqués aux sous-images annotées ;

E. d'un modèle de prédiction d'au moins une classe de microorganismes présents dans une lame en fonction de distributions spatiales des classes,

procédé selon lequel le prédicteur comprend la partie convolutive du premier réseau de neurones, en aval duquel est connectée le deuxième réseau de neurone, en aval duquel est connecté le modèle de prédiction.

Dans une variante de l'étape C, le procédé d'entrainement comprend l'entraînement d'un deuxième réseau de neurones comprenant une couche amont de pooling et une ou plusieurs couches aval de prédiction de la classe de microorganisme, le deuxième réseau ayant pour fonction de prédire la ou les classes d'une sous-image en fonction de vecteurs de caractéristiques produits par un extracteur de caractéristiques de patch, l'entrainement du deuxième réseau étant réalisé en fonction de vecteurs de caractéristiques en fonction des sous-images annotées de la base de données et des vecteurs de caractéristiques des patchs des sous-images annotés

En particulier, l'entrainement du premier réseau est réalisé sur les classes de microorganismes auxquels est ajoutée une classe « ambiguë », un patch annoté étant également annoté par cette classe lorsque des objets dans le patch en cas d'incertitude sur les objets présents dans le patch. En particulier, l'entrainement du deuxième réseau comprend l'entrainement d'un réseau MIL-CNN, la portion MIL entraînée du réseau MII,-CNN constituant le second réseau de neurones entraînés.

L'invention a également pour objet un procédé de prédiction d'une classe de microorganismes contenus dans un échantillon, parmi plusieurs classes de microorganismes, le procédé comprenant :

C. l'application, mise en oeuvre par ordinateur, d'un modèle de prédiction de la classe des microorganismes dans un échantillon étalé sur une lame en fonction d'une image acquise de ladite lame,

Selon l'invention, ladite image est subdivisée en sous-images et chaque sous-image est subdivisée en patchs, et l'application du modèle de prédiction comporte :

D. pour chaque patch, l'application d'un extracteur de caractéristiques de microorganismes, ledit extracteur comportant une partie convolutive d'un premier réseau de neurones convolutif, ledit premier réseau étant entraîné sur un ensemble de patchs d'entrainement comprenant des microorganismes, lesdits microorganismes étant annotés individuellement par au moins une classe ;

E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval de prédiction d'au moins une classe, sous la forme d'un score, ledit deuxième réseau étant entraîné sur des sous-images d'entrainement comprenant des microorganismes, chaque sous-image d'entrainement étant globalement annotée; et

F. pour l'image acquise :

a. le calcul d'un vecteur de caractéristiques en fonction des scores calculés pour les sous-images ;

b. l'application d'un modèle de prédiction d'au moins une classe pour les microorganismes présents dans l'échantillon en fonction du vecteur de caractéristiques, ledit modèle de prédiction étant entrainé sur des vecteurs de caractéristiques calculés à partir d'images d'entrainement.

L'invention a également pour objet un système de prédiction de prédiction d'une classe de microorganismes contenus dans un échantillon, parmi plusieurs classes de microorganismes, le système comprenant une unité informatique configurée pour mettre en oeuvre un modèle de prédiction de la classe des microorganismes dans un échantillon étalé sur une lame en fonction d'une image acquise de ladite lame. Selon l'invention, ladite image est subdivisée en sous-images et chaque sous-image est subdivisée en patchs, et l'application du modèle de prédiction comporte :

D. pour chaque patch, l'application d'un extracteur de caractéristiques de microorganismes, ledit extracteur comportant une partie convolutive d'un premier réseau de neurones convolutif, ledit premier réseau étant entraîné sur un ensemble de patchs d'entrainement comprenant des microorganismes, lesdits microorganismes étant annotés individuellement par au moins une classe ;
E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval de prédiction d'au moins une classe, sous la forme d'un score, ledit deuxième réseau étant entraîné sur des sous-images d'entrainement comprenant des microorganismes, chaque sous-image d'entrainement étant globalement annotée; et
F. pour l'image acquise :

a. le calcul d'un vecteur de caractéristique calculés pour les sous-images ;
b. l'application d'un modèle de prédiction d'au moins une classe pour les microorganismes présents dans l'échantillon en fonction du vecteur de caractéristiques, ledit modèle de prédiction étant entrainé sur des vecteurs de caractéristiques calculés à partir d'images d'entrainement.

L'invention a également pour objet des produit programme d'ordinateur comprenant une mémoire informatique mémorisant des instructions lisibles par ordinateur pour la mise en oeuvre des étapes D à F ci-dessus ou des étapes A à E ci-dessus.

L'invention a également pour objet un procédé de prédiction d'une classe d'objets contenu dans une image numérique parmi plusieurs classes d'objets, le procédé selon lequel l'image numérique est subdivisée en une pluralité de sous-images et chaque sous-image est subdivisée en patchs, et selon lequel :

D. pour chaque patch, l'application d'un extracteur de caractéristiques d'objets, ledit extracteur comportant une partie convolutive d'un premier réseau de neurones convolutif, ledit premier réseau étant entraîné sur un ensemble de patchs d'entrainement comprenant des objets, lesdits objets étant annotés individuellement par au moins une classe ;
E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval de prédiction d'au moins une classe, sous la forme d'un score, ledit deuxième réseau étant entraîné sur des sous-images d'entrainement comprenant des objets, chaque sous-image d'entrainement étant globalement annotée; et
F. pour l'image acquise :

a. le calcul d'un vecteur de caractéristique calculés pour les sous-images ;
b. l'application d'un modèle de prédiction d'au moins une classe pour les objets présents dans l'échantillon en fonction du vecteur de caractéristiques, ledit modèle de prédiction étant entrainé sur des vecteurs de caractéristiques calculés à partir d'images d'entrainement.

## BREVE DESCRIPTION DES FIGURES

L'invention sera mieux comprise à la lecture de la description qui suit, donnée uniquement à titre d'exemple, et réalisée en relation avec les dessins annexés, dans lesquels :

- la **figure 1** illustre les classes de microorganismes présentes sur une lame de Gram prédites par l'invention ;
- la **figure 2** est un organigramme d'un flux de travail de laboratoire d'analyse microbiologique mettant en oeuvre l'invention ;
- la **figure 3** est une vue schématique d'un système d'acquisition d'images de lames de Gram ;
- la **figure 4** est une illustration des différentes échelles d'une image de lame de Gram exploitée par l'invention ;
- la **figure 5** est un organigramme détaillant le fonctionnement du modèle prédiction à trois étage selon l'invention se fondant sur les différentes échelles de la figure 4 ;
- les **figure 6A-C** sont des vues schématique d extracteurs de caractéristiques de patch, comportant la partie convolutive d'un réseau de neurones convolutifs, dans cet exemple un réseau à architecture VGG16 ;
- la **figure 7** est une vue schématique d'un modèle prédictif au niveau sous-image, formé dans l'exemple de la partie MIL d'un réseau MIL-CNN à mécanisme d'attention ;
- la **figure 8** est une vue schématique du modèle prédictif au niveau image, se fondant sur un étage de prédiction du type Random Forest analysant une distribution de descripteurs fournis par l'étage de prédiction au niveau sous-image ;

- la **figure 9A** est une vue schématique illustrant l'affichage de zone présomptive de microorganisme dans l'image de la lame de Gram, ou dans une ou plusieurs sous-images constituant celle-ci en fonction des poids calculés par le mécanisme d'attention du modèle prédictif au niveau sous-image, et l'affichage des prédictions du modèle prédictif fonctionnant au niveau patch ;
- la **figure 9B** illustrent deux sous-images analysées par le réseau de neurones convolutifs dont est issu l'extracteur de caractéristique de patches constitutifs des sous-images, et les prédictions dudit réseau de neurones affichées sous la forme de « heat maps » ;
- la **figure 10** est un organigramme illustrant l'entrainement du modèle prédictif à trois étage selon l'invention ;
- les **figures 11A** et **11B** illustrent des performances de classification d'images de lame de Gram en imagerie RGB réalisée par le modèle prédictif à trois étages selon l'invention ;
- les **figures 12A** et **12B** illustrent des performances de classification d'images de lame de Gram en imagerie holographique réalisée par le modèle prédictif à trois étages selon l'invention ; et
- les **figures 13** à **15** illustrent différentes architectures informatiques pour la mise en oeuvre du modèle prédictif à trois étage selon l'invention et du flux de travail d'un laboratoire microbiologique exploitant ce dernier.

## DESCRIPTION DETAILLEE DE L'INVENTION

A. PROCEDE ET SYSTEME DE CARACTERISATION DU GRAM DE BACTERIES DANS UNE LAME DE GRAM

Il va à présent être décrit un mode de réalisation de l'invention, à savoir un flux de travail (ou « workflow ») de laboratoire microbiologique pour la caractérisation IVD notamment du Gram de bactéries chez un patient suspecté de septicémie, workflow se fondant sur l'analyse de l'image RGB d'une lame de Gram produite à partir d'une hémoculture positive.

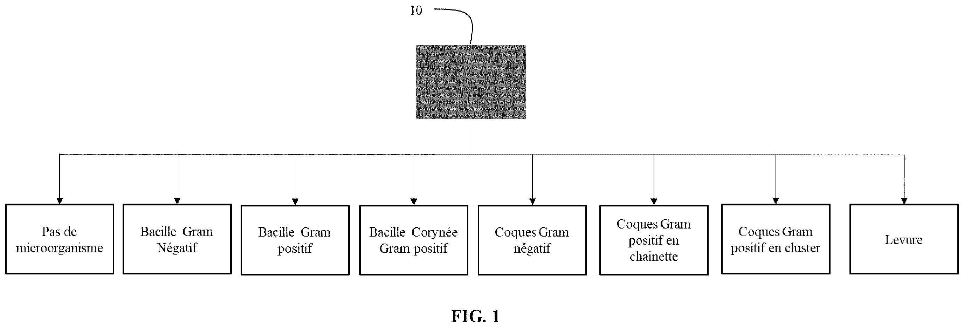

En particulier **(figure 1),** ce mode de réalisation comporte la classification automatique à plat d'une image de lame de Gram **10** en huit classes mutuellement exclusives, à savoir une lame ne comprenant ni bactérie ni levure, une lame comprenant des bacilles à Gram négatif, une lame comprenant des bacilles à Gram positif, une lame comprenant des bacilles corynée à Gram positif, une lame comprenant des coques à Gram négatif, une lame comprenant des coques à Gram positif en chaînette, une lame comprenant des coques à Gram positif en cluster et une lame comprenant des levures.

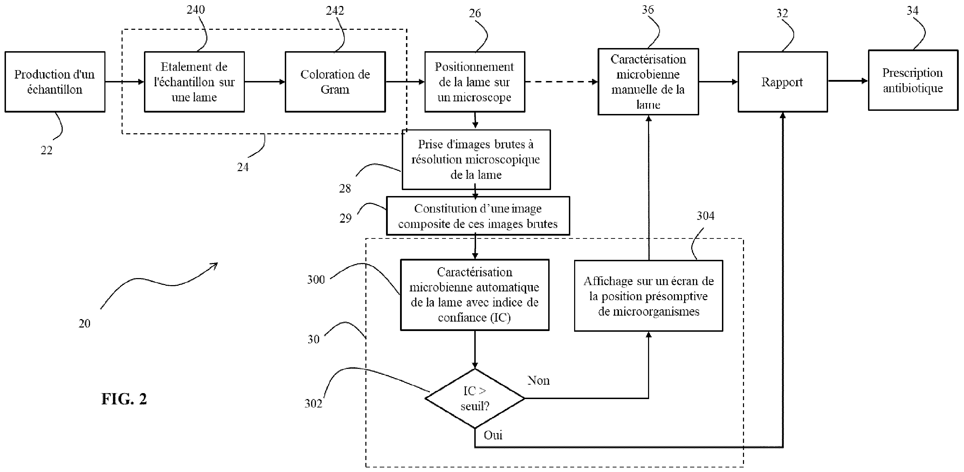

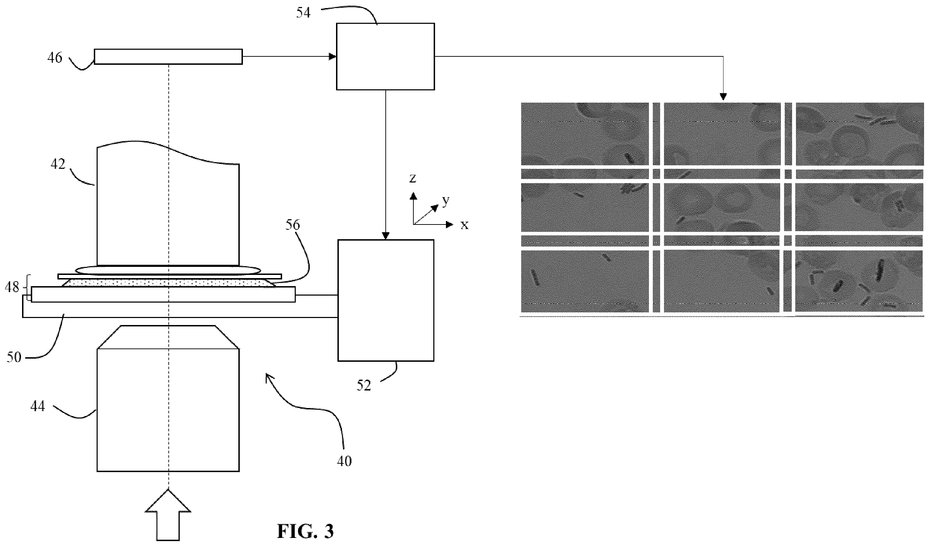

En se référant aux **figures 2** et **3,** ce workflow **20** débute par la production d'un échantillon **22** à partir d'un prélèvement de sang chez le patient, ici une hémoculture positive par exemple réalisée en utilisant un flacon de milieu BACT/ALERT® mis en culture dans le système BACT/ALERT® VIRTUO® de la demanderesse. Comme cela est connu en soit, l'hémoculture consiste à multiplier le nombre de bactéries et levures initialement contenues dans un prélèvement de sang afin de les rendre détectables (identification de l'état "présence" ou "absence" de bactéries ou de levure) et d'en faciliter leur caractérisation ultérieure en raison d'une biomasse plus importante. Pour ce faire le sang est mélangé à un milieu de culture par exemple à base de trypticase de soja, supplémentés de billes polymériques absorbantes. L'échantillon d'hémoculture est donc complexe en ce qu'il comprend les éléments naturellement présents dans le sang (hématies, globules blancs, plaquettes, fibrogènes...) ainsi que les éléments propres à l'hémoculture. Bien que le nombre de bactéries et levures a été multiplié, ces dernières peuvent malgré tout constituer une infime partie de l'échantillon et être masquées par, ou se confondre avec, des éléments hétérogènes notamment en nombre, forme, taille et colorimétrie.

Le procédé se poursuit, en **24,** par la production d'une lame de Gram à partir de l'hémoculture positive. Cette production comprend l'étalement ("smearing"), en **240,** d'une fraction de l'hémoculture de manière à obtenir une épaisseur d'étalement préférentiellement inférieure à $10\mu m$, cette épaisseur correspondant à la profondeur de champ d'un microscope avec un grossissement de 1000 utilisé ultérieurement dans le workflow. En ajustant l'épaisseur à ladite profondeur de champ, seule une inspection bidimensionnelle de l'étalement est nécessaire, facilitant de fait l'analyse telle que décrite ci-après. Une fois étalé, l'échantillon subit une coloration de Gram ("staining") telle que connue en soit, par exemple réalisée automatiquement au moyen de l'instrument PREVI® COLOR GRAM commercialisé par la Demanderesse, cette coloration ayant pour but de colorer de manière différente les bactéries en fonction de leur Gram.

Une fois séchée et recouverte d'une lamelle, la lame de Gram est positionnée, en **26,** dans un microscope **40** (figure 3) à objectif à immersion à huile de fort grossissement **42,** compris en 60x et 100x, couplé à système d'illumination **44** du type Köhler (illumination en lumière incohérente blanche dans au moins la gamme [400nm-900nm]), et à un système d'imagerie RGB **46**. Le système **46** comprend par exemple un capteur bidimensionnel de photosites CMOS, capteur recouvert d'une matrice de Bayer pour la production d'images en couleur d'une manière connue en soi, capteur placé dans le plan image de l'obj ectif **42**. Le système optique du microscope et le système d'imagerie sont choisis et/ou contrôlés de manière à ce que des bactéries et levures, objets de quelques centaines de nanomètres à quelques dizaines de micromètres, représentent au moins 5 pixels, de préférence au moins 10 pixels dans l'image obtenue.

Le champ de vision d'un tel objectif étant limité, la lame de Gram **48** est avantageusement placée sur un support mobile **50** déplaçable au moyen d'une platine à moteurs piézoélectriques **52,** permettant le déplacement de la lame dans le plan (x,y) perpendiculaire à l'axe optique z de l'objectif **42.** Cette platine est connectée à une unité informatique **54** également connectée au capteur **46,** unité informatique qui coordonne le déplacement du support **50,** et donc de la lame **48,** et la prise d'images par le capteur **46,** afin d'obtenir d'imager la totalité de la lame **48,** à tout le moins la totalité de la surface de lame sur laquelle est étalé l'échantillon **56.** De préférence, les images brutes acquises de la lame se recouvrent partiellement afin d'éviter des effets de bords, par exemple des bactéries, clusters ou chainettes de bactéries coupées.

La collection d'images peut être conservée pour former l'ensemble des « sous-images », cette collection correspond à une « image », telles que décrites ci-après, ou bien une seule image de la lame peut être obtenue par reconstruction informatique d'une manière connue en soi, ou bien cette collection d'images est redécoupée en « sous-images ». Selon l'invention, trois échelles sont obtenues pour une lame de Gram : une image (composite ou non), des sous-images constitutives de l'image, et des patchs constitutifs des sous-images. Cette collection d'images numériques, et de préférence chacune de ces images numériques, couvre une surface suffisamment importante de la lame pour être *a priori* représentative de la population de microorganismes présents dans l'échantillon initial, de telle sorte que la ou les classes de microorganismes présente(s) sur la lame soi(en)t a priori présente(s) quelque part sur l'image.

En se référant à nouveau à la **figure 2,** une image à résolution microscopique ou submicroscopique de la lame (dans cet exemple, une image composite de haute résolution, ou image « ICHR ») est donc produite à l'étape **28** du workflow. A titre d'illustration pour des lames de Gram classiquement utilisées en laboratoire, d'une dimension de 25mm par 75mm, il résulte une image d'au moins plusieurs dizaines de millions de pixels, avec le prototype des inventeurs 150 millions de pixels, avec une dimension latérale des pixels correspondant à 40nm, chaque pixel étant codés sur 16 bits pour chaque couleur. En particulier, le système d'acquisition **40, 46** produit quelques centaines de pixels pour un bacille de l'ordre du micromètre.

Le workflow de laboratoire **20** se poursuit par une étape automatique **30,** mise en oeuvre par ordinateur, d'analyse de l'image produite afin de caractériser au moins le Gram des bactéries présentes dans celle-ci, et plus spécifiquement prédire la ou les classes de la lame de Gram parmi les classes décrites précédemment. En particulier, une étape **300** de prédiction de type « multi-classes » ayant une architecture à trois étages telle que décrite ci-après est mise en oeuvre. Cette prédiction génère un vecteur dont les composantes correspondent aux probabilités des classes, à tout le moins des scores compris entre 0 et 1, dont la somme est égale à 1, ainsi qu'un indice de confiance ("*IC*") associé à la prédiction. La spécificité de la prédiction est optimisée de sorte que si l'indice *IC* dépasse un seuil de confiance prédéterminé (étape **302),** aucun autre test de caractérisation de la lame n'est nécessaire. Le résultat de la prédiction peut être ainsi directement poussé au clinicien (étape **32),** par exemple au moyen d'un rapport reçu par courriel ou au moyen d'une notification sur un smartphone ou équivalent, clinicien qui peut sur cette base choisir une antibiothérapie appropriée à administrer au patient (étape **34).**

De manière avantageuse, mais optionnelle, l'architecture de la prédiction comporte un mécanisme d'attention décrit ci-après. Outre une interprétabilité accrue de la prédiction, ce mécanisme permet d'identifier des zones de l'image de la lame de Gram susceptibles de contenir des microorganismes. Ainsi, là où le mécanisme a porté son attention, c'est-à-dit produit une pondération plus élevée, des zones présomptives de présence de microorganismes sont affichées, en **304,** sur un écran d'ordinateur en surimpression de l'image de la lame de Gram, à destination d'un technicien de laboratoire expert en interprétation de lame. Ce dernier, aidé par l'affichage, peut alors caractériser manuellement la lame (étape **36).** En option, ou en complément, le technicien peut également observer la lame directement au travers du microscope et caractériser cette dernière de manière classique. Notons que cet affichage peut être réalisé au niveau de l'image entière, ou bien décomposée, par exemple sur chacune des sous-images pour faciliter la lecture.

De manière avantageuse, mais optionnelle, l'architecture de la prédiction comporte également une étape intermédiaire de prédiction au niveau des patchs qui composent l'image et ses sous-images. Là encore, les prédictions des différents patchs peuvent être présentés en surimpression de l'image au technicien de laboratoire, ce qui permet non seulement d'orienter son attention sur les zones présomptives, mais également fournir des éléments présomptifs sur la classe des microorganismes présents dans les différentes zones de l'image. Là encore, une décomposition de l'affichage en sous-images est possible.

Comme décrit ci-après, le prototype développé par les inventeurs, entrainé sur moins de 600 lames, présente une précision macro de 95% pour un taux de rejet de lame de 17%. Très largement améliorable, ce prototype permet déjà d'automatiser la lecture de Gram d'une grande partie des échantillons d'hémoculture, tout en délivrant une information au-delà du simple Gram (bacille, coque, cluster, chaînette, levure...). Le temps gagné avec une telle automatisation pallie efficacement la raréfaction de l'expertise humaine dans ce domaine.

## B. ARCHITECTURE DE PREDICTION A MULTIPLES ETAGES POUR L'ANALYSE AUTOMATIQUE D'IMAGES COMPLEXES, EN PARTICULIER DE LAMES DE GRAM

Il va à présent être décrit plus en détail un mode de réalisation de l'architecture à étages d'un modèle prédictif (ou « prédicteur ») selon l'invention. Bien que cette architecture soit décrite en relation avec l'analyse d'une image RGB, cette architecture peut également être mise en oeuvre pour l'analyse d'images de nature différente, notamment holographiques comme cela sera décrit ci-après, ou encore multispectrales ou hyperspectrales.

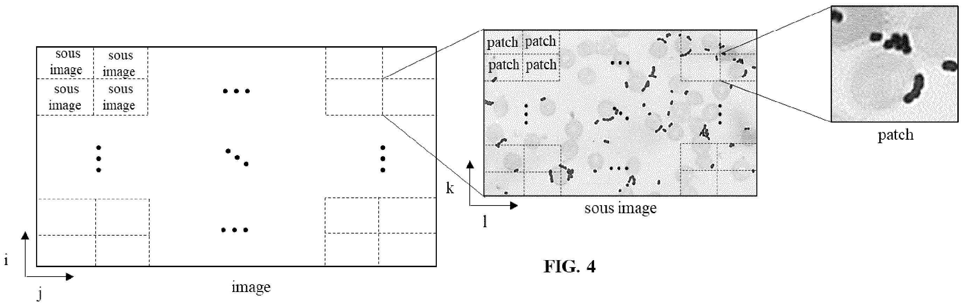

L'invention tire avantage de la très grande résolution de l'image ICHR, cette dernière pouvant être divisée en plusieurs dizaines, de préférence au moins une centaine, de sous-images, chaque sous-image pouvant être subdivisées en plusieurs dizaines de patchs, chaque patch ayant des dimensions en pixels suffisantes pour contenir un microorganisme ou un morphotype (cluster, chaînette...) dans sa globalité. Une telle subdivision est illustrée à la **figure 4** qui décrit une subdivision régulière de l'image en sous-images et des sous-images en patchs. En particulier sur la sous-image et le patch illustrés, des bacilles correspondent aux objets foncés, et sont de dimensions inférieures à celles du patch, et sont contenus entièrement dans le patch. Par exemple, les patchs ont une dimension de 256 pixels par 256 pixels (ou de 224 par 224) correspondant à une surface réelle d'environ $10 \times 10 \ \mu m^2$ alors que la dimension maximale typique des bactéries et levures est de l'ordre du micromètre, et les sous-images ont une dimension de 1024 pixels par 1536 pixels. Dans une variante privilégiée de l'invention, les patchs voisins se recouvrent partiellement afin d'éviter les effets de bords lors de l'entrainement du prédicteur au niveau patch. De manière similaire, les sous-images voisines se recouvrent partiellement pour la même raison. En variante, les sous-images ne couvrent pas la totalité de l'image ICHR et ne se recouvrent pas de manière à mieux couvrir la diversité liée à l'inhomogénéité du l'étalement.

Comme détaillé ci-après en relation avec l'apprentissage des étages du prédicteur multi-classe, cette subdivision, rendue possible par la très haute résolution de l'image ICHR, est associée à une annotation de force décroissante depuis les patchs vers l'image ICHR. En particulier, pour une base de données d'images ICHR d'apprentissage subdivisées en sous-images et chaque sous-image en patchs :

    i. des patchs sont annotés de manière forte pour chaque sous-image à minima par la ou les classes des microorganismes qu'il contient. En particulier, un patch annoté est avantageusement, mais optionnellement, associé à une double annotation : une première annotation globale correspondant à la ou les classes des microorganismes qu'il contient ainsi qu'une seconde annotation pour chacun de ses pixels codant la présence ou absence de microorganisme ;

    ii. chaque sous-image est annotée globalement par la ou les classes des microorganismes présents;

    iii. chaque image est globalement annotée par la ou les classes des microorganismes présents.

Selon l'invention, un modèle extrait des caractéristiques des patchs par un modèle de prédiction de classe au niveau patch, caractéristiques de patchs qui sont transmise à un deuxième étage qui extrait des caractéristiques des sous-images par un modèle de prédiction de classe au niveau sous-image, caractéristiques de sous-image qui sont transmises à un troisième étage qui réalise une prédiction de classe finale des microorganismes présents dans l'image, et donc dans la lame de Gram.

Dans ce qui suit, une image ICHR est subdivisée en une matrice bi-dimensionnelle de sous-images référencées par les indices *(i, j)* et chaque sous-images est subdivisées par une matrices bi-dimensionnelle de patchs référencés par les indices *(k, l)*.

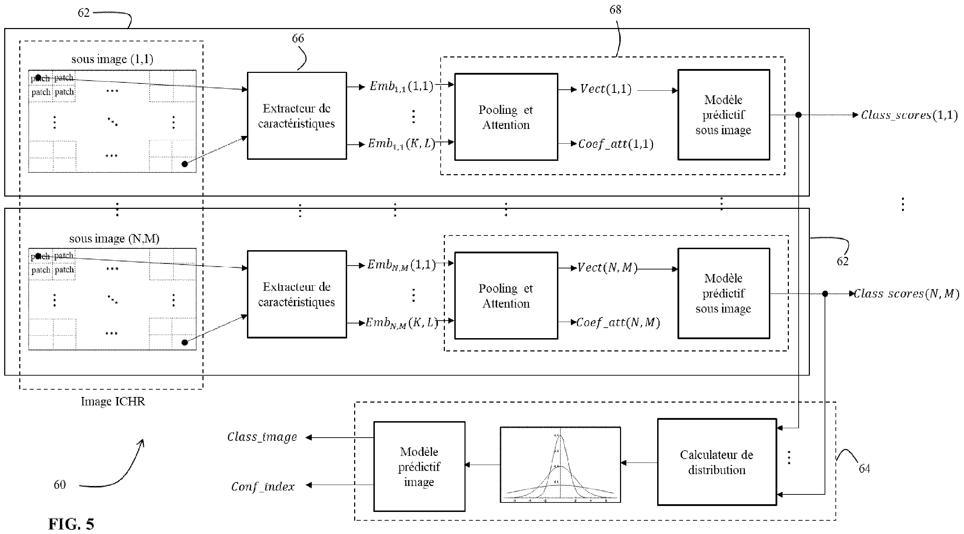

En se référant à la **figure 5,** un mode de réalisation particulier du prédicteur **60** comprend :

    A. un flux d'analyse **62** pour chaque sous-image d'une image ICHR, chaque flux produisant un vecteur de scores, un score par classe, (noté « *Class_scores(i, j)* » pour la sous-image de coordonnée *(i, j)*). Les flux d'analyse **62** sont réalisés de manière indépendante et sont identiques en termes de modules computationnels ;

    B. un flux d'analyse globale **64** recevant les vecteurs de caractéristiques de chaque flux **62,** dans une variante privilégiée illustrée à la **figure 5** les vecteurs de scores, et produisant un vecteur de scores, un score par classe, pour l'image ICHR, noté « *Class_image* »*,* ainsi qu'un indice de confiance pour cette prédiction, noté « *Conf_index* ».

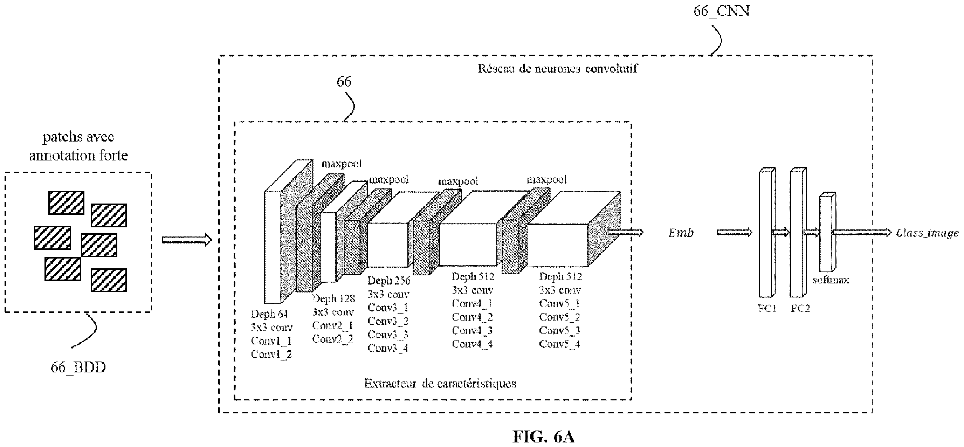

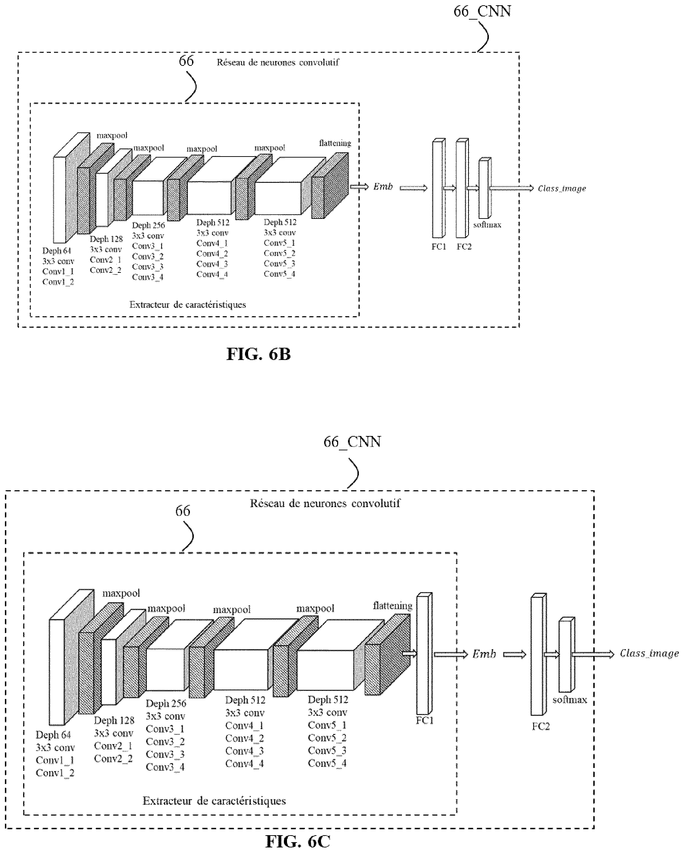

Chaque flux **62** d'analyse d'une sous-image comporte un extracteur de caractéristiques **66** constitué préférentiellement de la partie convolutive d'un réseau de neurones convolutif **(figure 6A).** En variante, l'extracteur comporte la partie convolutive d'un réseau de neurones, suivie d'une couche d'aplatissement (« flattening ») **(figure 6B).** En variante, l'extracteur comporte la partie convolutive d'un réseau de neurones, suivie d'une couche d'aplatissement suivi d'une ou plusieurs couches pleinement connectées **(figure 6C).** Optionnellement, ces variantes sont complétées en aval par une ou plusieurs couches de neurones pleinement connectées, le vecteur de caractéristiques correspondant à la sortie de la dernière couche connectée.

Cet extracteur a pour fonction d'extraire des caractéristiques de chaque patch constitutif de la sous-image, notées « *Emb* », qui résument l'information contenue dans les patchs, notamment en termes de présence ou d'absence de microorganismes.

En se référant à la **figure 6A,** ce réseau de neurones convolutif **66_CNN** est entrainé sur une base de données de patchs d'entrainement **66_BDD** annotés, le réseau **66_CNN** ayant pour fonction de prédire la ou les classes de microorganismes présents dans les patchs. Dans un mode de réalisation privilégié de l'invention, le réseau de neurones **66_CNN** est un réseau de type VGG16, ResNet, MobileNetV2, ou efficientNet, de préférence pré-entraîné sur des bases de données publiques tel que disponibles à l'URL https://www.image-net.org, en particulier un réseau VGG16 entraîné sur la base d'ImageNet « ImageNet Large Scale Visual Recognition Challenge (ILSVRC) » (https://www.image-net.org/ challenges/LSVRC/index.php). Bien qu'il soit possible de prendre un réseau vierge, c'est-à-dire initialisé par des poids aléatoires, et l'entrainer *ab initio* avec des patchs annotés, l'utilisation d'un réseau pré-entraîné permet une augmentation de la précision macro de la prédiction de la ou des classes de l'image ICHR de plusieurs pourcents. Parmi les dizaines de réseaux pré-entraînés testés par les inventeurs, un VGG16 est le plus performant, menant à une augmentation de la précision macro d'environ 5% par rapport à un réseau entraîné uniquement à des patchs d'images de lame. S'agissant d'un réseau à l'architecture figée, la dimension des patchs issus des images ICHR est choisie pour être identique. Cette dimension satisfait la condition sur les dimensions des microorganismes, bactéries et levures, telle que décrite plus haut.

Dans une variante avantageuse mais optionnelle, les prédictions rendues par le réseau de neurones convolutif **66_CNN** peuvent être utilisées pour identifier les zones des sous-images où telle ou telle classe est probablement présente. Ces résultats peuvent être présentés sous forme de « heat map » et sur-imposées à l'image. Une sous-partie **66** du réseau **66_CNN** est ensuite extraite, avec ses paramètres. Cette partie **66** peut comprendre les couches convolutives de **66_CNN,** et éventuellement certaines des couches pleinement connectées situées en aval. On peut également, optionnellement, y ajouter en aval de nouvelles couches pleinement connectées. Cet élément **66** fonctionne comme un extracteur de caractéristiques des patchs, caractéristiques pertinentes pour la prédiction des classes concernées.

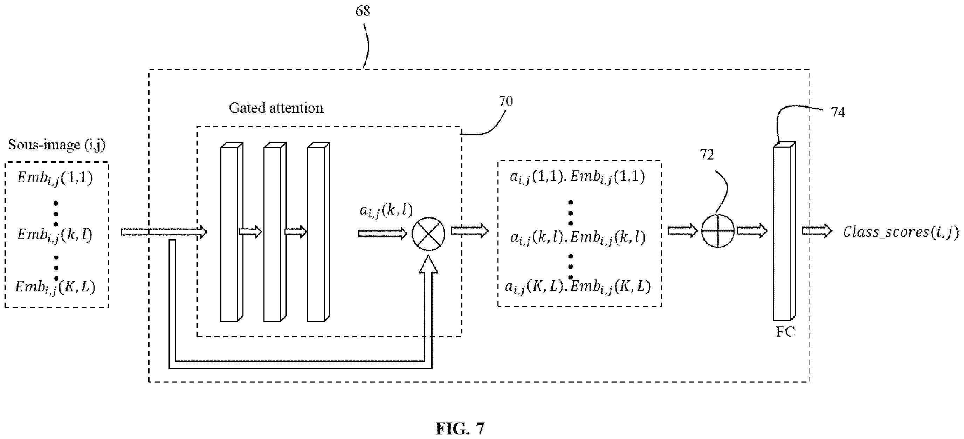

Une fois les caractéristiques extraites de chaque patch, les vecteurs de caractéristiques correspondants sont communiqués à un deuxième étage de prédiction **68** à base de réseaux de neurones mettant en oeuvre une prédiction multi-classes de la ou les classes de microorganisme présents dans la sous-image constituée des patchs. Plus spécifiquement, comme illustré à la **figure 7,** ce deuxième étage **68** est la portion « MIL» (pour « *Multiple Instance Learning* ») d'un réseau de type MIL-CNN dont la partie extracteur de caractéristiques est issue du modèle **66_CNN,** avantageusement l'extracteur **66.**

Le deuxième prédicteur **68** comporte :

i. un étage amont **70** mettant en oeuvre un mécanisme d'attention à porte (en anglais « *gated attention mechanism* »). Cet étage **70** calcule pour chaque vecteur de caractéristiques issu des patchs (noté $Emb_{i,j}(k, l)$ pour le patch de coordonnées $(k, l)$ dans la sous-image de coordonnées $(i, j)$), un coefficient $a_{i,j}(k, l)$ mesurant le poids du patch dans la prédiction de classe de la sous-image, poids que l'étage **70** multiplie aux vecteurs correspondant pour produire un nouveau vecteur de caractéristique $a_{i,j}(k, l) \times Emb_{i,j}(k, l)$ pour chaque patch. Dans un mode de réalisation préféré, mais non obligatoire, ce poids est calculé selon la relation :

$$a_{i,j}(k, l) = \frac{exp\left(W^T\left(tanh(VEmb_{i,j}(k, l)^T) \odot sigm(UEmb_{i,j}(k, l)^T)\right)\right)}{\sum_{i,j} exp\left(W^T\left(tanh(VEmb_{i,j}(k, l)^T) \odot sigm(UEmb_{i,j}(k, l)^T)\right)\right)}$$

Où $U \in \mathbb{R}^{Q \times R}$ et $W \in \mathbb{R}^Q$ sont des matrices formant paramètres du prédicteur **68,** Q étant la dimension des vecteurs de caractéristiques $Emb_{i,j}(k, l)$, R un entier positif prédéterminé, par exemple égal à 512, *sigm* la fonction non-linéaire sigmoïde, et $\odot$ est l'opérateur de multiplication terme à terme. Un tel mécanisme est notamment décrit dans l'article de M. Ilse et al., « Attention-based Deep Multiple Instance Learning », arXiv:1802.04712v4 [cs.LG], 28 Jun 2018. D'autres mécanismes d'attention sont cependant possibles.

ii. en aval de l'étage à mécanisme d'attention **70,** ou intégré à ce dernier, un étage de pooling **72** réduisant la dimensionnalité globale des vecteurs de caractéristiques. Par exemple, l'étage **72** produit pour chaque sous-image *(i, j)* un vecteur unique de caractéristiques $Z(i,j)$ à partir des $K \times L$ vecteurs issus des patchs selon la relation :

$$Z(i, j) = \sum_{k,l=(1,1)}^{k,l=(K,L)} a_{i,j}(k, l) \times Emb_{i,j}(k, l)$$

iii. en aval de l'étage de pooling **72,** un étage à couches de neurones pleinement connectés **74,** recevant le vecteur

$Z(i,j)$ en entrée et produisant en sortie un vecteur de scores de prédiction $Class\_scores(i, j)$. Les scores sont normalisés, compris entre 0 et 1, et par convention, plus un score est élevé, plus la probabilité que la classe correspondante soit présente dans la sous-image est élevée. Dans la modalité considérée, cet étage aval peut se réduire à une couche de type sigmoïde qui produit les scores, mais si besoin d'autres couches peuvent être insérées entre l'étage de pooling et l'étage d'obtention des scores.

Dans un mode de réalisation privilégié, le modèle **66** et sa partie aval **68** se trouvent ainsi intégrés dans un nouveau prédicteur qui fonctionne au niveau-sous-image, et qui est celui mis en oeuvre dans le flux de travail **62** de la **figure 5**. Comme décrit dans l'article de Ilse et al., ce nouveau prédicteur peut être appris sur des exemples de sous-images, notamment par des techniques classiques de rétropropagation. Préférentiellement, mais optionnellement, on laissera les paramètres de la partie **66** du modèle libre d'évoluer au cours de cet apprentissage, de sorte qu'à la fin de ce processus ceux-ci n'auront plus leur valeur issue de l'apprentissage du modèle **66_CNN**. Par ce procédé, on co-optimise l'extracteur de caractéristique **66** et les couches aval **68**.

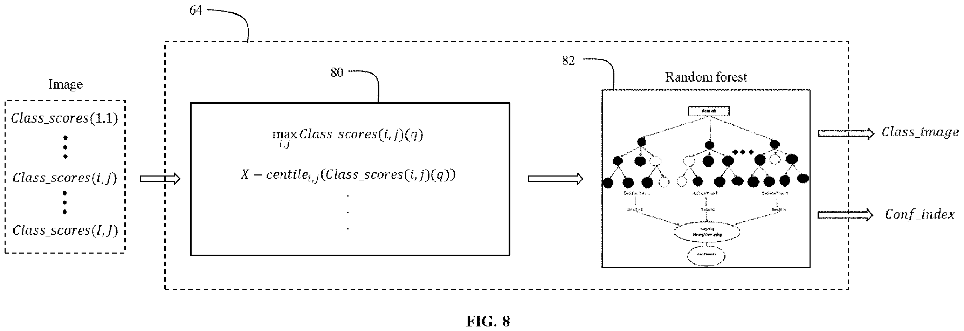

En se référant à la **figure 8,** le dernier étage **64** du prédicteur **60** comporte :

i. un étage amont **80** recevant des descripteurs, issus par exemple des vecteurs de scores de prédiction $Class\_scores$ $(i, j)$ de l'ensemble des sous-images constitutives de l'image de la lame de Gram, et calculant une distribution des scores sur la lame pour chacune des classes à partir de ces vecteurs. Cet étage **80** tire avantage du nombre important de sous-images constituant l'image ICHR en raison de la grande résolution de cette dernière. Plus particulièrement, l'image ICHR étant constituée de plusieurs dizaines, voire une centaine ou plus, de sous-images, il est ainsi possible de calculer de manière pertinente statistiquement une distribution des scores desdites classes présentes dans l'image ICHR. Un avantage de calculer une distribution est d'obtenir une caractérisation de l'image ICHR indépendante de la position des microorganismes dans l'image tout en prenant en compte l'ensemble de l'image. En particulier, pour des applications aussi sensibles que le diagnostic in vitro de patient, notamment pour le sepsis, il est douteux de rendre un résultat se fondant sur une zone unique ou limitée de la lame. En effet, la prédiction basée sur une seule sous-image peut être erronée ou bien trop incertaine.

Par exemple, pour chaque classe de microorganisme, l'étage **80** calcule une approximation de cette distribution consistant en l'extraction des statistiques suivantes

- le maximum des scores
- le 95$^{ieme}$ percentile
- le score médian
- le 5$^{ieme}$ percentile

Cette approximation est rapide à calculer et permet de régler, au travers de chacune de ses composantes les performances globales de la prédiction. Notamment, le maximum ainsi que le 95$^{ième}$ percentile permet de régler le niveau de sensibilité de la prédiction des classes de microorganismes effectivement présents (ici le 95$^{ième}$ percentile est choisi, mais d'autres valeurs supérieures à 50% sont possibles en fonction de la sensibilité voulue). La valeur médiane permet d'atténuer les erreurs de prédiction commises au niveau sous-image. Le 5$^{ième}$ percentile permet de régler la sensibilité de la prédiction concernant la classe « pas de microorganisme » (ici le 95$^{ième}$ percentile est choisi, mais d'autres valeurs inférieures à 50% sont possibles en fonction de la sensibilité voulue). On notera que des approximations de distribution plus simples sont également dans la portée de l'invention (par exemple uniquement le score maximal pour chaque classe) comme des approximations plus complexes (comme par exemple une interpolation polynomiales des scores ou encore l'approximation par une distribution caractérisée par ses équations tels que la loi de Fisher, de Gauss, de Bernouilli...). En particulier, l'avantage d'obtenir des statistiques descriptives est de pouvoir tenir compte des spécificités de l'application, connues de l'expert en microbiologie, comme l'illustre le choix des percentiles pour aider à régler la sensibilité et la sensibilité qui sont deux critères importants dans le diagnostic IVD.

ii. un étage aval **82** recevant chacune des distributions des classes et le nombre de sous-images $I \times J$, et prédisant en sortie la ou les classes $Class\_image$ de microorganismes présents dans l'image ICHR ainsi qu'un indice de confiance $Conf\_index$ de cette prédiction. Plus particulièrement, la prédiction mise en oeuvre par l'étage **82** est une prédiction multi-classes par apprentissage automatisé, et de préférence une prédiction ne mettant pas en oeuvre de réseau de neurones. En effet, les caractéristiques reçues par cet étage sont structurées avec un nombre déterminé, fixe et de nature connue de caractéristiques. Aussi, les approches « classiques » d'apprentissage automatisé (i.e. non à base de réseau de neurones), telles que les approches à base de SVM (« support vector machine »), des K plus proches voisins, d'arbres de décision pour en citer quelques-unes, sont plus adaptées en termes de performances, d'interprétabilité et de facilité d'entrainement. Parmi l'ensemble de ces approches, l'étage **82** met en oeuvre de manière préférentielle une prédiction de type « Random Forest » particulièrement efficace pour éviter le sur-apprentissage et pour délivrer un indice de confiance *IC* (par exemple égal au maximum des pourcentage de votes pour chaque classe) interprétable. En effet, comme l'ont remarqué les inventeurs pour l'interprétation d'images complexes, l'indice de confiance du Random Forest prend une valeur élevée pour les prédictions précises (i.e. conformes à la réalité des microorganismes présents dans la lame) et s'effondre dans le cas contraire. Le choix d'une valeur seuil telle qu'utilisée à l'étape **302** (figure 2) pour une application aussi sensible qu'un diagnostic in vitro est ainsi facilité et robuste. Le modèle prédictif de type Random Forest est par exemple celui décrit à l'article de Breiman et al., "Random Forests", Machine Learning, 45(1), 5-32, 2001.

Notons que les descripteurs reçus par le modèle **80** ne sont pas nécessairement issus des scores de prédiction rendus par le prédicteur **68**. En particulier, il est possible d'exploiter des descripteurs issus des couches situés plus en amont dans son architecture.

Les différents modèles prédictifs (niveau patch, sous-image, ou image) décrits ci-avant sont des modèles prédictifs « multi-classes ». En variante, il est possible pour le modèle patch et/ou le modèle sous-image et/ou le modèle image de mettre en oeuvre ici une prédiction « multi-étiquette », c'est-à-dire de type présence/absence pour chacune des classes considérées, et non présence d'une seulement des classes (la classe négative étant alors traitée comme une classe à part). La prédiction multi-étiquette permet de traiter certains cas particuliers, tel celui d'échantillons poly-microbiens présentant plusieurs types de Gram. L'avantage de choisir des modèles prédictif multi-classes est de réduire le nombre de données annotées d'entraînement et/ou la campagne de collecte de lames de Gram ou d'échantillons correspondant. En effet, dans le cadre d'infections microbiennes, le cas poly-microbien par exemple est largement minoritaire par rapport aux infections mono-microbiennes, de sorte qu'il existe beaucoup moins de données associées, ce qui rend plus difficile l'entraînement des modèles prédictifs multi-étiquettes.

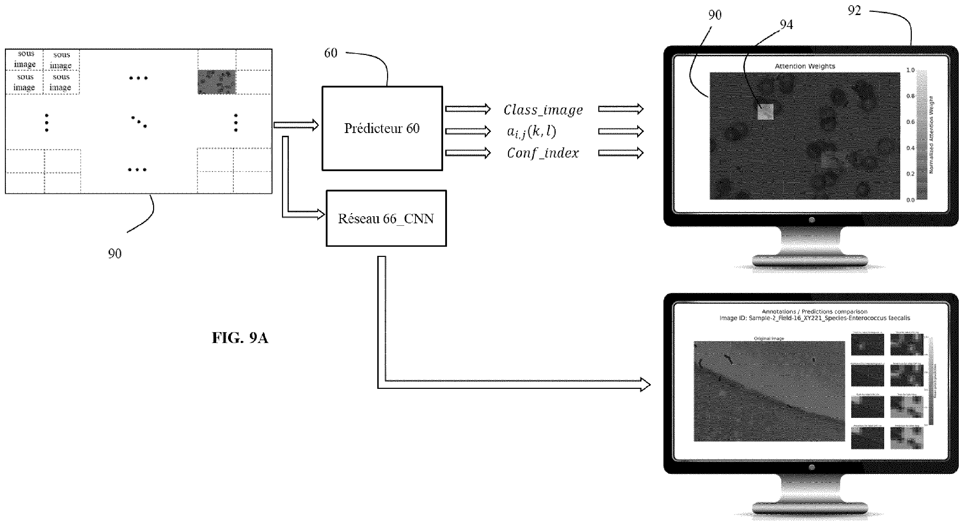

En se référant à la **figure 9A,** une utilisation privilégiée des résultats en sortie du prédicteur **60** faisant suite à l'analyse d'une image de lame **90** par ce dernier comporte l'affichage sur un écran d'ordinateur **92** de l'image **90** dont l'intensité lumineuse est réglée patch par patch en fonction des coefficients $a_{i,j}(k, l)$. Notamment, plus le poids d'un patch est élevé, signifiant son importance élevée dans la prédiction au niveau sous-image, plus le patch est lumineux, comme illustré par le patch **94** bien plus clair que les autres zones de l'image **90.** De cette manière, un technicien de laboratoire peut, s'il le souhaite, vérifier le contenu de ce patch en termes de microorganismes présents et confirmer ou infirmer son contenu. Le technicien peut ainsi afficher l'ensemble de l'image ou, préférentiellement pour des raisons de lisibilité, une sous-image particulière telle qu'illustré dans cette figure. De préférence, sont également affichés la ou des classes prédites, codée dans le vecteur *Class_image,* l'indice de confiance associée *Conf_index,* et un signal codant l'échec de la prédiction lors que l'indice est inférieur au seuil. Les patchs plus lumineux font alors office de zones présomptives susceptibles de contenir des microorganismes. Cette aide permet alors au technicien de plus rapidement réaliser son analyse en se focalisant sur ces zones en premier.

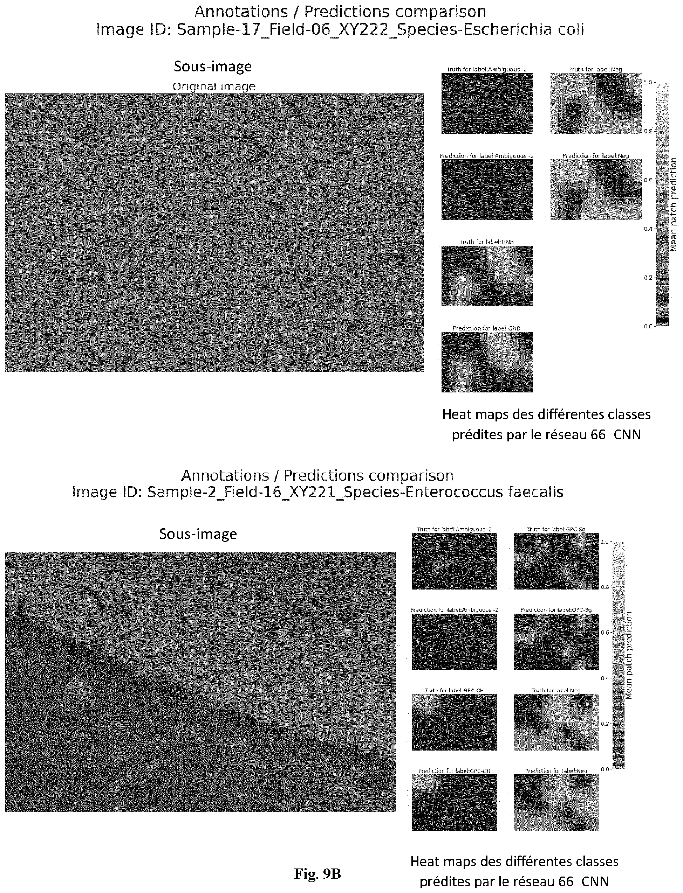

De manière avantageuse, comme illustré sur le deuxième écran d'affichage de la **figure 9A** et les images de la **figure 9B,** les sous-images sont également analysées par le réseau de neurones convolutifs **66_CNN** dont les prédictions peuvent être affichées sur un écran, avantageusement sous forme de « heat maps », de sorte que le technicien peut directement savoir quelles zones de l'image sont susceptibles de correspondre à des microorganismes, et à quelle classe ceux-ci correspondent.

C. ANNOTATION DES DONNEES ET ENTRAINEMENT DE L'ARCHITECTURE DE PREDICTION A MULTIPLES ETAGES

C. 1. ANNOTATION DES DONNEES

Trois échelles d'annotation sont réalisées : au niveau de l'image entière, à laquelle correspond la lame ; au niveau de la sous-image ; et au niveau du patch.

Considérons d'abord le niveau lame, qui est le niveau pertinent d'un point de vue biologique et médical. Chaque image couvre *a priori* une surface suffisamment grande pour rendre compte du contenu de la lame, et de l'échantillon initial. Aussi on peut utiliser toute connaissance disponible sur le contenu de l'échantillon ou de la lame pour produire une annotation au niveau image.

De préférence, les lames de Gram sont issues de laboratoires d'analyse microbiologique, réalisées et annotées en conditions réelles par des techniciens spécialistes de l'analyse de Gram. Toutefois, l'interprétation des lames complexes peut s'avérer difficile, même pour un technicien expérimenté. De préférence, une partie de l'échantillon servant à la réalisation d'une lame est caractérisé de manière plus approfondie. Notamment, l'identité réelle des microorganismes présents dans l'échantillon est déterminée, de préférence par spectrométrie de masse de type MALDI-TOF, au moyen d'un VITEK® MS commercialisé par la Demanderesse. L'image de la lame de Gram hérite tout d'abord de l'annotation de la lame faite en laboratoire, qualifiée de « brute ». Cette annotation brute est alors vérifiée par un second expert et les erreurs et ambiguïtés d'annotation corrigées grâce aux caractérisations supplémentaires des microorganismes. L'annotation finale de l'image de la lame est alors qualifiée de « vérité terrain ». Outre la correction de l'annotation brute, un premier tri des lames est réalisé : celles comprenant un mélange polymicrobien ou des *Campylobacter* sont écartés du jeu d'entrainement. Afin d'améliorer les performances globales du prédicteur selon l'invention, les *Acinetobacter* sont classées dans la classe « Bacille à Gram Négatif ». D'autres caractéristiques des échantillons (métadonnées) sont également collectées comme, le cas échéant, le type de milieu de culture compris dans l'échantillon, le temps écoulé pour obtenir la positivité de l'hémoculture, le temps écoulé pour la révélation de la coloration de Gram, ou encore un antibiogramme des microorganismes.

Décrivons maintenant l'annotation au niveau sous-image. Chaque sous-image d'une image reçoit à défaut l'annotation « vérité terrain » de cette dernière, mais cette annotation peut être modifiée par le second expert, par exemple dans le cas où le microorganisme présent sur la lame est absent de la sous-image considérée. Pour ce faire, pour caractériser l'image de la lame dans son entier, le second expert parcourt chaque zone de celle-ci et donc chaque sous-image. De manière préférentielle et optionnelle, lorsque le second expert n'arrive pas à annoter de manière non ambigüe une sous-image, cette dernière est écartée du jeu d'entrainement. De manière préférentielle et optionnelle, l'annotation « vérité terrain » des sous-images est modifiée et réduite à une seule classe, de sorte qu'on pourra se restreindre ensuite à un modèle de type « multi-classes », les rares sous-images présentant simultanément deux classes étant exclues du jeu d'entraînement. De manière préférentielle et optionnelle, le cas particulier de bactéries présentant plusieurs morphes (par exemple les Bacilles Gram Variables comme *Bacillus subtilis,* qui est taxonomiquement un Bacille Gram Positifs, mais qui souvent présente l'apparence de Bacilles Gram Négatifs), l'annotation au niveau sous-image pourra être modifiée de manière à rendre compte de l'apparence réelle des bactéries, de telle sorte que l'annotation sous-image peut se trouver différente de l'annotation image. On obtient ainsi une annotation pour tout ou partie des sous-images, qui sont typiquement une centaine de fois plus nombreuses que le nombre d'images dans la base. Les sous-images sont annotées de manière forte puisque le concepteur du prédicteur selon l'invention injecte une connaissance, donc une information *a priori* supplémentaire, dans l'annotation de la sous-image. Cette annotation servira à améliorer l'entraînement du prédicteur.

Selon la même logique, une annotation plus forte encore peut être produite au niveau patch, sur une partie au moins du jeu de données. Dans un mode de réalisation de l'invention, cette annotation est réalisée en effectuant d'abord une segmentation sémantique des microorganismes, c'est-à-dire en associant un type de Gram à chacun des pixels d'une sous-image. Divers procédés sont possibles pour réaliser cette étape de segmentation sémantique. On peut par exemple effectuer une segmentation semi-automatisée via un outil comme Ilastik décrit dans l'article de S. Berg et al. « ilastik: interactive machine learning for (bio)image analysis », Nature Methods, (2019) et disponible à l'URL https://www.ilastik. org/, associant un statut de microorganisme ou non à chaque pixel. On peut ensuite utiliser l'annotation de la sous-image pour associer une classe aux pixels de microorganismes. Une dernière étape manuelle effectuée par un expert permet enfin de corriger les masques de segmentation obtenue, et également de pointer des zones ambiguës. Une fois la segmentation sémantique effectuée, un algorithme explicite permet d'attribuer à chacun des patchs de la sous-image une classe, en fonction du nombre de pixels de la classe en question présents sur le patch.

De même que l'annotation sous-image n'est pas nécessairement identique à l'annotation image, l'annotation patch n'est pas nécessairement identique à l'annotation sous-image. En particulier, lorsque certaines classes sont liées à l'état d'organisation des microorganismes (chaînettes, grappes), une telle organisation n'est pas toujours détectable au niveau d'un patch d'étendue réduite, aussi dans le cas où le nombre de pixels de microorganismes n'est pas suffisamment grand sur un patch, il peut convertir l'annotation de telle sorte qu'elle ne rende plus compte de l'état d'agrégation. Par exemple, on convertira par ce procédé la classe « Coque Gram Positif Agrégé en Chaînette » vers « Coque Gram Positif - état d'agrégation indéterminable ». Enfin, une nouvelle classe « ambiguë » peut être attribuée à un patch soit lorsque des pixels qui le composent ont été annotés comme tel par un expert, soit quand le nombre de pixels associés à un microorganisme est très faible (ce qui correspond en général à un organisme vu partiellement car situé en bord de patch).

Une fois ce procédé mis en oeuvre, on obtient une annotation au niveau patch pour tout ou partie du jeu de données disponible. Notons que le nombre de patchs, dans la modalité considérée, est typiquement de l'ordre de quelques dizaines ou centaines par sous-image. Les patchs sont annotés de manière très forte puisque le concepteur du prédicteur selon l'invention injecte une information *a priori* supplémentaire dans l'annotation du patch. Cette annotation servira à améliorer l'entraînement du prédicteur.

Les images des lames sont stockées avec leur annotation « vérité terrain » dans une mémoire informatique pour leur traitement ultérieur. De même, les sous-images et leur annotation sont stockées dans une mémoire informatique. De même, les patchs avec leurs annotations de patch sont stockés dans une mémoire informatique pour leur traitement ultérieur. Trois bases de données sont ainsi constituées.

On obtient par le procédé décrit ci-dessus une annotation à trois niveaux croissants de force : niveau image, sous-image et patch. Ce faisant, on renforce considérablement la force globale de notre annotation sur le jeu d'apprentissage. Or, étant donné le nombre d'images disponibles (737 dans l'exemple de mise en oeuvre considéré) et leur dimensionnalité (plus de 100 millions de pixels dans l'exemple considéré), l'entraînement direct d'un prédicteur exploitant uniquement l'annotation niveau image serait très vraisemblablement voué à l'échec.

## C.2. ENTRAINEMENT DU PREDICTEUR A MULTIPLE ETAGES

Il est à présent décrit un procédé d'entrainement du prédicteur **60.** Selon ce procédé, chaque étage est entraîné indépendamment des autres tout en se fondant sur les mêmes images de lame de Gram d'entrainement.

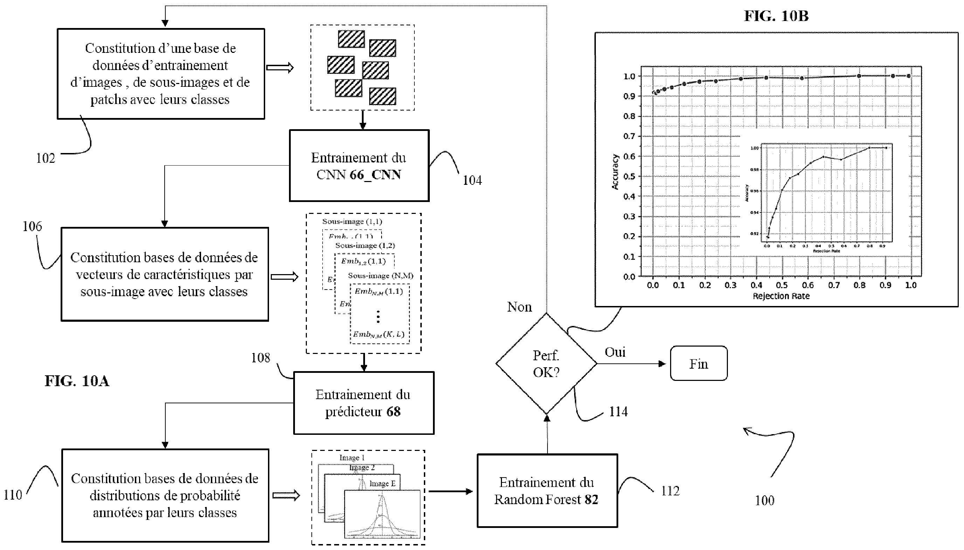

En se référant à la **figure 10A,** un procédé **100** d'entrainement du prédicteur **60** comprend la constitution, en **102,** d'une base de données images de lame de Gram d'entrainement, annotées par la ou les classes de microorganismes présents, notamment une base de données d'images annotés, de sous-images annotées et de patchs annotés de la manière décrite précédemment.

Le procédé se poursuit par l'entrainement, en **104,** du réseau de neurones convolutifs **66_CNN** à prédire les classes de patchs. Cet entraînement peut débuter par un réseau non entraîné. Cependant comme décrit précédemment, un réseau initial pré-entraîné est préférentiellement sélectionné puis réentraîné sur la base de données de patchs annotés. Dans cette option, en fonction du réseau pré-entraîné choisi, une normalisation de la valeur des pixels des patchs peut être mise en oeuvre. Notamment, certains réseaux accessibles dans des bibliothèques de réseaux, notamment ceux se fondant sur les bibliothèques logicielles « TensorFlow », sont entraînés sur images dont les valeurs de pixels sont standardisées. Dans ce cas, en amont du réseau, **66_CNN** il est prévu une normalisation des patchs, par exemple une standardisation (centrage des données sur 0, et de l'écart-type à 1). Cet étage de normalisation est alors optionnellement prévu dans le prédicteur selon l'invention, en amont de l'extracteur de caractéristiques **66.**

La base de données de patchs annotés est scindée en un jeu d'entrainement et un jeu de test, en faisant en sorte (technique dite de « blocking ») que tous les patchs issus d'une image donnée soient soit tous versés dans le jeu d'entraînement, soit versés dans le jeu de test. L'optimisation des hyperparamètres du réseau **66_CNN** est réalisée sur le jeu d'entraînement par une technique de validation croisée, par exemple 4 « folds » constitués en utilisant la technique dite de « blocking » (ou « blocked cross-validation »), qui garantit notamment que tous les patchs associés à une image donnée sont positionnés dans le même « fold ». La valeur des hyperparamètres est par exemple optimisée selon une technique de recherche par grille (« grid search »), de recherche aléatoire (« random search »), ou par une méthode bayésienne.

Dans une variante, les patchs ayant été annotés « ambigus » sont conservés pour l'entraînement et le test, et l'apprentissage comprend une classe supplémentaire « ambiguë », cette classe regroupant les patchs pour lequel l'expert annotant n'est pas certains des microorganismes présents.

Une fois le réseau CNN **66_CNN** entrainé, la partie convolutive est extraite, les dernières couches étant supprimées du réseau.

Dans une première variante de l'apprentissage du modèle prédictif du niveau sous-image, l'extracteur est intégré à un modèle de type MIL-CNN par un procédé de *transfer learning.* Ce réseau est par exemple celui décrit dans l'article de M. Ilse et al. Par exemple le code source, ainsi que le code source d'entrainement, du réseau sont ceux accessibles sur Github à l'adresse https://github.com/AMLab-Amsterdam/AttentionDeepMIL. Le procédé se poursuit, en **108,** par l'entrainement du réseau MIL-CNN, la base de sous-images étant scindée en un jeu d'entraînement et un jeu de test, en faisant en sorte d'avoir la même répartition des images entre jeu d'entraînement et test que pour l'entraînement du modèle patch. La valeur de ses hyperparamètres est optimisée là aussi par validation croisée, par exemple selon une technique de recherche par grille (« grid search »), de recherche aléatoire (« random search »), ou par méthode bayésienne. De manière préférentielle, ces hyperparamètres comprennent l'architecture de l'étage à couche de neurones pleinement connectés **74** (e.g. nombre de couches, nombre de neurones par couche...). Dans cette variante, la partie convolutive et la partie MIL du modèle MIL-CNN entraîné peuvent constituer respectivement l'extracteur **66** au niveau patch et le modèle de prédiction **68** au niveau sous-image. En variante, la portion MIL est extraite pour former le modèle **68** et l'extracteur est celui entraîné à l'étape précédente.

Dans une deuxième variante d'apprentissage du modèle prédictif au niveau sous-image, seule la portion MIL d'un réseau MIL-CNN est entraînée. Par exemple, l'ensemble des patchs de toutes les sous-images sont traités par l'extracteur entraîné **66** de manière à obtenir des ensembles de vecteurs de caractéristiques correspondants, chacun annoté par l'annotation de la sous-image correspondante comme illustrée à la **figure 10A.** Ces jeux de vecteurs de caractéristiques et leurs annotations sont mémorisés dans une base de données **106.** Le modèle **68** est alors entraîné sur cette base selon une technique de cross-validation telle que décrite précédemment.

Une fois l'étage MIL **68** entrainé, chaque lot de sous-images correspondant à une image est traité, en **110,** par cet étage **68** et le module **80** de calcul de distribution afin de produire des distributions de scores, distributions qui sont mémorisées avec les annotations d'image correspondante dans une base de données. Puis, en **112,** le dernier étage **82** du prédicteur à base de Random Forest est entraîné, la base de distributions étant scindée en un jeu d'entrainement et un jeu de test, là encore avec la même répartition des lames que précédemment., L'optimisation des hyperparamètres est là encore réalisée par validation croisée avec 4 folds. La valeur de ses hyperparamètres est par exemple optimisée selon une technique de recherche par grille (« grid search »), le nombre d'hyperparamètres étant plus limité que dans le cadre des prédicteurs précédents.

De manière optionnelle, le procédé se poursuit, en **114,** par le calcul d'un critère de performance du prédicteur à étage selon l'invention, par exemple la précision globale, en fonction d'un taux de rejet des lames. Notamment, ce calcul à traiter l'ensemble des images de lame par le prédicteur et de faire varier le seuil de confiance de l'étape **302** (figure 2). Les lames ne passant pas le seuil sont écartées, le taux de rejet étant égal au pourcentage de lames écartées, et la précision globale est calculée pour les lames restantes. Selon l'invention, si le taux de rejet est jugé trop important ou la précision trop faible, le procédé se poursuit par l'acquisition de nouvelles lames et/ou l'annotation supplémentaire de patchs et le réentrainement du prédicteur tel que décrit précédemment afin de diminuer le taux de rejet et d'augmenter la précision.

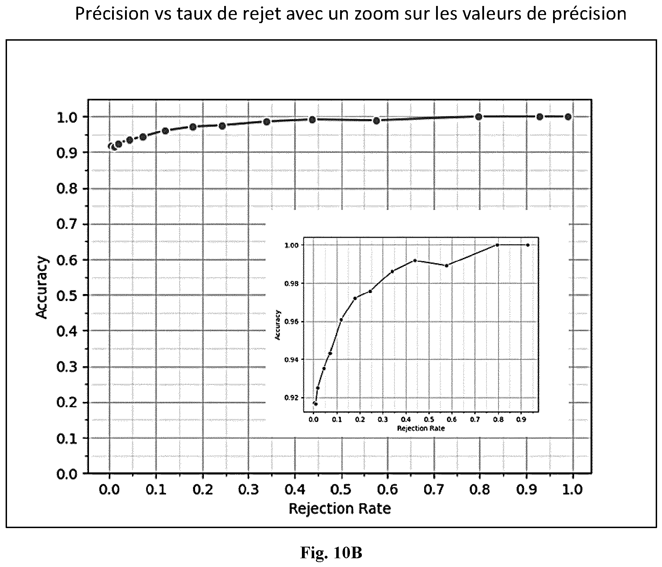

La figure **10B** illustre un exemple de précision vs taux de rejet pour le prototype de prédicteur à étage conçu par les inventeurs après plusieurs itérations. Sur cet exemple, en ciblant une précision supérieure ou égale à 95%, le taux de rejet obtenu est de 17%, signifiant que ce prototype, si utilisé en l'état en combinaison avec le système d'acquisition d'image décrit à la figure 3, permet de traiter de manière automatique, sans test supplémentaire, plus de 80% des lames de Gram. Ces performances ont été obtenues sur un jeu de données initial de 737 lames de Gram, divisées chacune en 100 sous-images, chaque sous-image étant divisée en 24 patchs. Sur nombre total de patchs de près de 1,8 millions, seuls 3% de ceux-ci, soit environ 50 000, ont été annotés de manière forte, alors que le nombre total de paramètres du prédicteur selon l'invention est d'environ 15 millions.

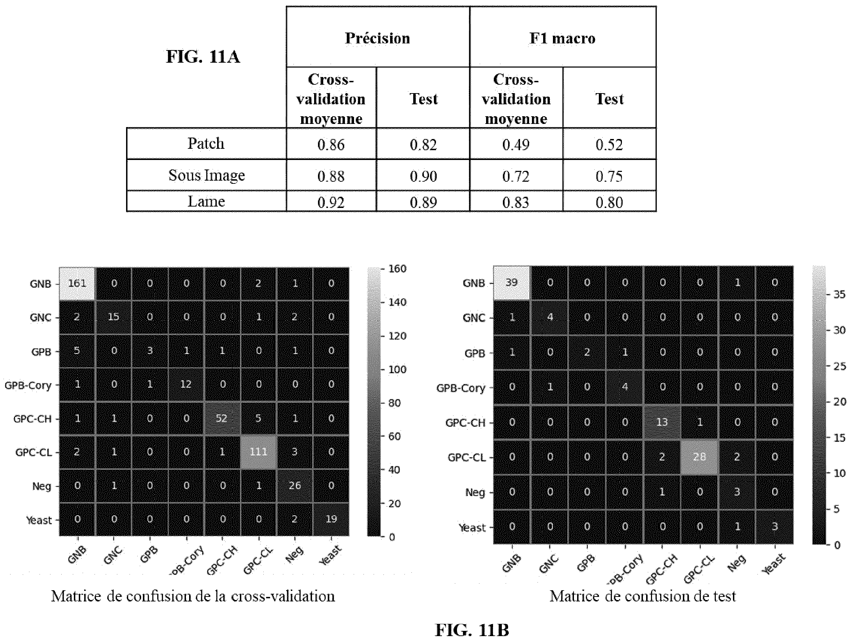

La figure **11A** présente avec plus de détails les performances obtenues par le prototype du prédicteur selon l'invention, entrainé sur le jeu de lame de Gram précédemment décrit, performances obtenues sur l'ensemble des lame (i.e. avec un taux de rejet réglé à 0%). La figure **11B** présente la matrice de confusion correspondante.

## D. APPLICATION DE L'INVENTION A DES SIGNAUX AUTRES QUE DES SIGNAUX RGB

Il a été décrit une application de l'invention à des images dont la valeur des pixels est codée sur trois canaux de couleur (RGB). L'invention s'applique à d'autres types de signaux, notamment des images numériques dont la valeur des pixels code des informations holographiques, sur une ou plusieurs longueurs d'onde.

Par exemple, le système d'acquisition de l'image de la lame de Gram est celui décrit dans les demandes de brevet EP4307051 et EP4172699, connecté à un module de calcul pour la reconstruction d'une image refocalisée. Indépendamment des variantes particulières décrites dans ces demandes, le principe de l'imagerie holographique est d'éclairer en lumière cohérente selon une ou plusieurs longueurs d'onde la lame, d'enregistrer les images d'intensité correspondantes et de produire par reconstruction informatique (reconstruction dite « paramétrique » comme par exemple décrit dans ces demandes ou reconstruction dite « non paramétrique» comme décrit par exemple dans les demandes WO2016075279, WO2017077238 ou WO17207184) une image numérique holographique de la lame dont chaque pixel est codé par une valeur d'intensité et une valeur de phase pour chaque longueur d'onde d'éclairage (dans un mode de réalisation décrit dans les deux demandes EP4307051 et EP4172699, au nombre de huit, chaque pixel étant ainsi codé sur 16 canaux).

Dans une première variante, l'extracteur de caractéristique **66** est issu d'un réseau convolution **66_CNN** qui n'est pas pré-entraîné, ce qui permet de prendre en entrée l'ensemble des canaux. Dans une seconde variante, cet extracteur est issu d'un réseau convolutif pré-entraîné, notamment sur des images RGB, tel que décrit précédemment. Dans cette option, en amont de l'extracteur **66,** il est prévu un étage de réduction de dimensionnalité si le nombre de canaux par pixel est supérieur à 3. De manière avantageuse, cet étage consiste à calculer 3 composantes principales de l'image holographique et d'injecter ces trois composantes principales dans l'extracteur. De manière avantageuse, les composantes principales choisies pour la réduction de dimensionnalité sont des hyperparamètres lors de l'entraînement du réseau convolutif **66 CNN**

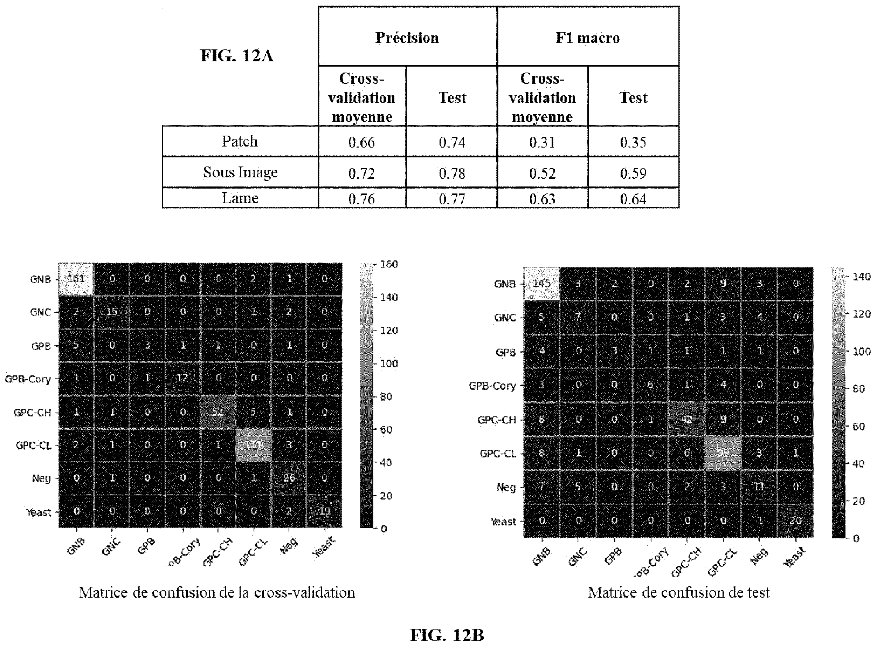

Les annotations des images de lame, des sous-images et des patchs, ainsi que l'entrainement du prédicteur, sont réalisées de manière analogue à la manière décrites ci-avant Les **figures 12A** et **12B** décrivent les performances du prototype à image holographique développé par les inventeurs, performances obtenues sur la base initiale des 737 lames de Gram.

## E. EXTENSION DE L'ENSEIGNEMENT DES MODES DE REALISATION DETAILLES

i. Il a été décrit l'application de l'invention à la caractérisation de microorganismes dans un échantillon préparé en lame de Gram. L'invention s'applique à tout type d'image numérique comprenant des objets à caractériser. Par exemple, en restant dans le domaine de la biologie, cette technique s'applique à l'imagerie cellulaire de cellules eucaryotes, en particulier de cellules marquées par fluorescence dont les différents types peuvent être détectés, ainsi que leurs différents organites, tel le noyau cellulaire. Un autre exemple est l'hématologie, où les différents éléments figurés du sang (érythrocytes, leucocytes) peuvent être détectés. Un troisième exemple est l'analyse d'échantillons d'urine, avec là encore les éléments figurés peuvent être détectés conjointement à des microorganismes. Un quatrième exemple est l'histopathologie, pour détecter des cellules cancéreuses qui peuvent ne couvrir qu'une très petite partie de la surface d'une image de très haute résolution.

Pour certaines de ces applications où la notion de quantification ou de numération est importante (ce qui n'est pas le cas sur les hémocultures positives), l'analyse du nombre de patchs et/ou de sous-images positifs à telle ou telle classe peut constituer un point de départ pour mettre en oeuvre un comptage des différents types cellulaires.

D'une manière générale, l'invention trouve application à la caractérisation de tout objet dans une image de haute résolution, composite ou non.

ii. Il a été décrit un mode de réalisation dans lequel les caractéristiques fournies au dernier étage sont les scores de prédictions du second étage. En variante, les caractéristiques sont par exemple celles générées par une couche de neurones de la portion MIL. Selon l'invention, des statistiques descriptives sont également générées à partir de ces caractéristiques et communiquées au modèle prédictif final.

iii. Il a été décrit un modèle prédictif au niveau sous-image mettant en oeuvre un mécanisme d'attention. En variante, ce modèle ne met pas en oeuvre cette fonction, les vecteurs de caractéristiques issus des patchs étant directement groupés via une méthode de pooling telle l'application d'une moyenne ou d'un max pooling.

## F. MISE EN ŒUVRE INFORMATIQUE

La phase d'entrainement du prédicteur de l'invention, notamment celle décrite en relation avec la figure **10A** et la phase de prédiction, notamment celle décrite en relation avec la **figure 5,** hormis les étapes de préparation de la lame et d'acquisition du signal optique correspondant à l'image de celle-ci, sont mises en oeuvre par ordinateur, à savoir au moyen de circuits matériels comprenant des mémoires informatiques (cache, RAM, ROM...) et un ou plusieurs microprocesseurs ou processeurs (CPU et/ou GPU), organisés ou non sous forme de noeuds de calcul, nécessaires à l'exécution d'instructions informatiques mémorisées dans les mémoires pour la mise en oeuvre desdites phases. On comprendra que s'agissant de calcul tout type d'architecture informatique peut convenir et que la description ci-avant et ci-après ne doit pas être comprise comme limitant la portée de l'invention.

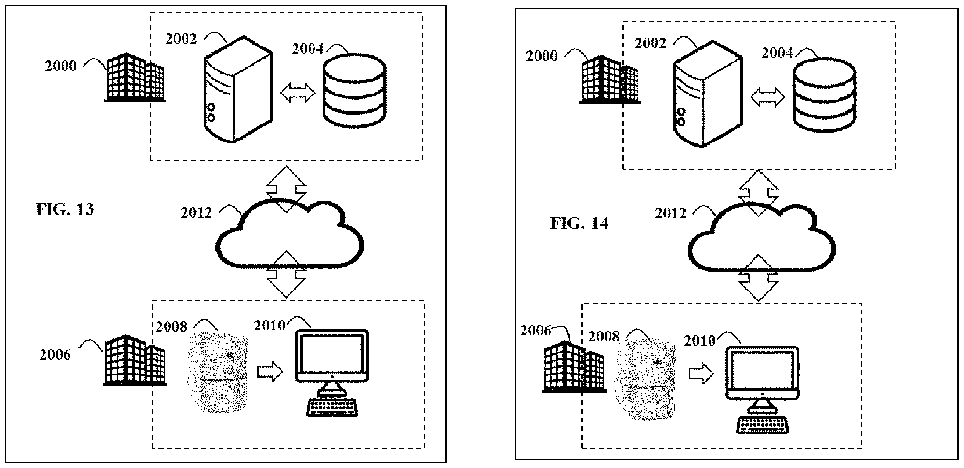

Plusieurs architectures sont possibles, comme par exemple illustrées aux **figures 13-15.**

Dans une première variante d'architecture **(fig. 13),** une première organisation **2000,** par exemple la Demanderesse, héberge ou contrôle un ou plusieurs serveurs de calcul **2002** associés à une ou plusieurs bases de données **2002** pour la mémorisation des images de lames annotées et la phase d'apprentissage est mise en oeuvre par l'organisation **2000** sur son ou ses serveurs **2002.** Une seconde organisation **2006,** par exemple un laboratoire microbiologique, héberge un microscope **2008** tel que décrit ci-avant, connecté à, ou incorporant, un ordinateur personnel, un ordinateur de bureau ou un serveur **2010** et met en oeuvre la préparation de la lame de Gram jusqu'à l'acquisition de l'image numérique de la lame mémorisé par l'unité informatique **2010.** Cette image est alors poussée, au travers d'un réseau de connexion à distance, à la première organisation **2000** pour la mise en oeuvre sur le serveur **2002** (ou une unité informatique différente de celle utilisée pour l'entrainement, par exemple mise en oeuvre sur un Cloud sous la forme du Software As a Service) du reste de la phase de prédiction. Le compte rendu de la classification des microorganismes présents, ou non, produit est alors poussé, au travers du réseau **2012,** vers la seconde organisation **2006** qui prend ou non des mesures thérapeutiques en fonction du compte rendu.

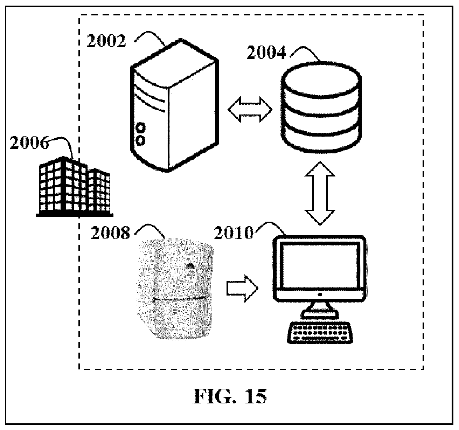

Une seconde variante d'architecture **(fig. 14)** diffère de la première, en ce qu'une copie du logiciel mettant en oeuvre la phase de prédiction sont téléchargées dans la seconde organisation qui met en oeuvre à l'aide de l'ordinateur **2010** ou d'un serveur de calcul (non représenté) la totalité de la phase de prédiction. Dans une variante de cette architecture, ce téléchargement correspond à une copie du logiciel dans l'ordinateur **2010,** ordinateur fourni par la première organisation **2000.** Dans une troisième variante **(fig. 15),** la totalité des phases d'apprentissage et de prédiction sont mises en oeuvre par une seule organisation **2006.**

## Revendications

1. Procédé de prédiction d'une classe de microorganismes contenus dans un échantillon, parmi plusieurs classes de microorganismes, le procédé comprenant :

   A. la préparation d'une lame, en particulier de microscope, comprenant l'étalement de l'échantillon sur ladite lame ;
   B. l'acquisition d'au moins une image numérique de la lame avec une résolution micrométrique ou sub-micrométrique;

C. l'application, mise en oeuvre par ordinateur, d'un modèle de prédiction de la classe des microorganismes en fonction de l'image acquise,

*caractérisé en ce que* ladite image est subdivisée en une pluralité de sous-images et chaque sous-image est subdivisée en patchs, *et en ce que* l'application du modèle de prédiction comporte :

D. pour chaque patch, l'application d'un extracteur de caractéristiques de microorganismes, ledit extracteur comportant une partie convolutive d'un premier réseau de neurones convolutif, ledit premier réseau étant entraîné sur un ensemble de patchs d'entrainement comprenant des microorganismes, lesdits microorganismes étant annotés individuellement par au moins une classe ;

E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval comprenant une couche mettant en oeuvre une prédiction d'au moins une classe, sous la forme d'un score, ledit deuxième réseau étant entraîné sur des sous-images d'entrainement comprenant des microorganismes, chaque sous-image d'entrainement étant globalement annotée; et

F. pour l'image acquise :

a. le calcul d'un vecteur de caractéristique calculés pour les sous-images ;

b. l'application d'un modèle de prédiction d'au moins une classe pour les microorganismes présents dans l'échantillon en fonction du vecteur de caractéristiques, ledit modèle de prédiction étant entrainé sur des vecteurs de caractéristiques calculés à partir d'images d'entrainement.

**2.** Procédé selon la revendication 1, *caractérisé en ce que* le vecteur de caractéristiques est une distribution des scores calculés pour les sous-images.

**3.** Procédé selon l'une des revendications 1 ou 2, *caractérisé en ce que* chaque image d'entrainement est subdivisée en sous-images et chacune desdites sous-images est subdivisée en patchs, *et en ce que* moins de 50% des patchs des images d'entrainement sont annotés, lesdits patchs annotés formant les patchs d'entrainement du premier réseau convolutif.

**4.** Procédé selon la revendication 3, *caractérisé en ce que* moins de 10% des patchs des images d'entrainement sont annotés.

**5.** Procédé selon l'une quelconque des revendications précédentes, *caractérisé en ce que* l'entrainement des premier et second réseaux convolutifs et du modèle de prédiction est configuré pour obtenir une spécificité macro de prédiction supérieure ou égale à 90%

**6.** Procédé selon l'une quelconque des revendications précédentes, *caractérisé en ce que* le modèle de prédiction de l'étape F est un modèle « random forest ».

**7.** Procédé selon l'une quelconque des revendications précédentes, *caractérisé en ce que* la couche amont de pooling est associée à une couche d'attention configurée pour appliquer un poids à la sortie de chaque extracteur.

**8.** Procédé selon l'une quelconque des revendications précédentes, *caractérisé en ce que* le deuxième réseau est un réseau MII,-CNN entrainé par lots d'instances, les lots d'instances étant constitués des patchs d'entrainement.

**9.** Procédé selon l'une quelconque des revendications précédentes, *caractérisé en ce que* le vecteur de caractéristiques de l'étape F.a comporte pour chaque classe :

- le score maximal parmi les sous-images ; et/ou
- le $X^{ième}$ percentile des scores parmi les sous-images, avec X supérieur ou égal à 90%, de préférence égal à 95% ; et/ou
- le $Y^{ème}$ percentile des scores parmi les sous-images, avec Y inférieur ou égal à 10%, de préférence égal à 5% ; et/ou
- le score médian parmi les sous-images.

**10.** Procédé selon la revendication 8, *caractérisé en ce qu'*il comprend en outre le nombre de sous-images dans l'image.

**11.** Procédé selon l'une quelconque des revendications précédentes, *caractérisé en ce que* le premier réseau convolutif est pré-entraîné sur des images ne comprenant pas de microorganismes puis entraîné sur des patchs d'entrainement annotés.

**12.** Procédé selon la revendication 10, *caractérisé en ce que* le premier réseau convolutif pré-entrainé est un réseau VVG16 ou ResNet.

**13.** Procédé selon l'une quelconque des revendications précédentes, *caractérisé en ce que* les microorganismes comprennent des bactéries et les classes comprennent au moins le Gram positif et le Gram négatif, *et en ce que* la préparation de la lame comprend la préparation d'une lame de Gram.

**14.** Procédé selon la revendication 13, *caractérisé en ce que* les classes de microorganismes comprennent en outre des classes de morphotypes.

**15.** Procédé selon l'une quelconque des revendications précédentes, *caractérisé en ce que* les classes de microorganismes comprennent une classe « ni bactérie ni levure », une classe « bacilles à Gram négatif », une classe « bacilles à Gram positif », une classe « bacilles corynée à Gram positif », une classe « coques à Gram négatif », une classe « coques à Gram positif en chaînette », une classe « coques à Gram positif en cluster » et une classe « levures ».

**16.** Procédé selon l'une quelconque des revendications précédentes, *caractérisé en ce que* l'échantillon comprend du sang.

**17.** Procédé selon l'une quelconque des revendications précédentes, *caractérisé* en ce que l'échantillon est une hémoculture positive.

**18.** Procédé d'entrainement d'un modèle de prédiction d'une classe de microorganismes parmi plusieurs classes de microorganismes à partir d'une image numérique d'une lame sur laquelle est étalé un échantillon susceptible de comprendre des microorganismes, ledit procédé d'entraînement comprenant :

A. la constitution d'une base de données d'entrainement comprenant des images numériques de lame annotées par une ou plusieurs classes de microorganismes, les images des lames étant divisées en une pluralité de sous-images annotées par une ou plusieurs classes de microorganismes et les sous-images d'entrainement étant subdivisée en patchs, au moins une partie des patchs étant annotés par une ou plusieurs classes de microorganismes,
B. l'entraînement d'un premier réseau de neurones convolutifs en fonction des patchs annotés ;
C. l'entraînement d'un deuxième réseau de neurones à partir de la base annotée de sous-images, ledit second réseau comprenant au moins un extracteur de caractéristiques de patchs, un étage de pooling des caractéristiques de patchs, et un étage de prédiction de la ou les classes de microorganismes présents sur la sous-image;
D. la constitution d'une base de données de distributions des classes présentes dans les lames d'entrainement en fonction des classes prédites par le deuxième réseau de neurones appliqués aux sous-images annotées ;
E. d'un modèle de prédiction d'au moins une classe de microorganismes présents dans une lame en fonction de distributions spatiales des classes,

procédé selon lequel le prédicteur comprend la partie convolutive du premier réseau de neurones, en aval duquel est connectée le deuxième réseau de neurone, en aval duquel est connecté le modèle de prédiction.

**19.** Procédé d'entrainement selon la revendication 18, *caractérisé en ce que* l'entrainement du premier réseau est réalisé sur les classes de microorganismes auxquels est ajoutée une classe « ambiguë », un patch annoté étant également annoté par cette classe lorsque des objets dans le patch en cas d'incertitude sur les objets présents dans le patch.

**20.** Procédé d'entrainement selon la revendication 18 ou 19, *caractérisé en ce que* l'entrainement du deuxième réseau comprend l'entrainement d'un réseau MII,-CNN, la portion MIL entraînée du réseau MIL-CNN constituant le second réseau de neurones entraînés.

**21.** Procédé de prédiction d'une classe de microorganismes contenus dans un échantillon, parmi plusieurs classes de microorganismes, le procédé comprenant :

C. l'application, mise en oeuvre par ordinateur, d'un modèle de prédiction de la classe des microorganismes dans un échantillon étalé sur une lame en fonction d'une image acquise de ladite lame,

***caractérisé en ce que*** ladite image est subdivisée en sous-images et chaque sous-image est subdivisée en patchs, ***et en ce que*** l'application du modèle de prédiction comporte :

D. pour chaque patch, l'application d'un extracteur de caractéristiques de microorganismes, ledit extracteur comportant une partie convolutive d'un premier réseau de neurones convolutif, ledit premier réseau étant entraîné sur un ensemble de patchs d'entrainement comprenant des microorganismes, lesdits microorganismes étant annotés individuellement par au moins une classe ;

E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval de prédiction d'au moins une classe, sous la forme d'un score, ledit deuxième réseau étant entraîné sur des sous-images d'entrainement comprenant des microorganismes, chaque sous-image d'entrainement étant globalement annotée par au moins une classe ; et

F. pour l'image acquise :

a. le calcul d'un vecteur de caractéristiques en fonction des scores calculés pour les sous-images ;

b. l'application d'un modèle de prédiction d'au moins une classe pour les microorganismes présents dans l'échantillon en fonction du vecteur de caractéristiques, ledit modèle de prédiction étant entrainé sur des vecteurs de caractéristiques calculés à partir d'images d'entrainement.

22. Procédé de prédiction selon la revendication 21, ***caractérisé en ce que*** les étapes D à F sont conformes à l'une quelconque des revendications 2 à 17.

23. Système de prédiction de prédiction d'une classe de microorganismes contenus dans un échantillon, parmi plusieurs classes de microorganismes, le système comprenant une unité informatique configurée pour mettre en oeuvre un modèle de prédiction de la classe des microorganismes dans un échantillon étalé sur une lame en fonction d'une image acquise de ladite lame,

***caractérisé en ce que*** ladite image est subdivisée en sous-images et chaque sous-image est subdivisée en patchs, ***et en ce que*** l'application du modèle de prédiction comporte :

D. pour chaque patch, l'application d'un extracteur de caractéristiques de microorganismes, ledit extracteur comportant une partie convolutive d'un premier réseau de neurones convolutif, ledit premier réseau étant entraîné sur un ensemble de patchs d'entrainement comprenant des microorganismes, lesdits microorganismes étant annotés individuellement par au moins une classe ;

E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval de prédiction d'au moins une classe, sous la forme d'un score, ledit deuxième réseau étant entraîné sur des sous-images d'entrainement comprenant des microorganismes, chaque sous-image d'entrainement étant globalement annotée par au moins une classe ; et

F. pour l'image acquise :

a. le calcul d'un vecteur de caractéristique calculés pour les sous-images ;

b. l'application d'un modèle de prédiction d'au moins une classe pour les microorganismes présents dans l'échantillon en fonction du vecteur de caractéristiques, ledit modèle de prédiction étant entrainé sur des vecteurs de caractéristiques calculés à partir d'images d'entrainement.

24. Système de prédiction selon la revendication 23, ***caractérisé en ce que*** l'unité informatique est configurée pour mettre en oeuvre des étapes D à F sont conformes à l'une quelconque des revendications 2 à 17.

25. Produit programme d'ordinateur comprenant une mémoire informatique mémorisant des instructions lisibles par ordinateur pour la mise en oeuvre des étapes D à F selon l'une quelconque des revendications 2 à 17.

26. Produit programme d'ordinateur comprenant une mémoire informatique mémorisant des instructions lisibles par ordinateur pour la mise en oeuvre des étapes A à E selon l'une quelconque des revendications 18 à 20.

27. Procédé de prédiction d'une classe d'objets contenu dans une image numérique parmi plusieurs classes d'objets, le procédé selon lequel l'image numérique est subdivisée en une pluralité de sous-images et chaque sous-image est subdivisée en patchs, et selon lequel :

D. pour chaque patch, l'application d'un extracteur de caractéristiques d'objets, ledit extracteur comportant une partie convolutive d'un premier réseau de neurones convolutif, ledit premier réseau étant entraîné sur un ensemble de patchs d'entrainement comprenant des objets, lesdits objets étant annotés individuellement par au moins une classe ;

E. pour chaque sous-image, l'application d'un deuxième réseau de neurones, connecté pour recevoir les caractéristiques extraites des patchs constitutifs de ladite sous-image, ledit deuxième réseau comprenant une couche amont de pooling et une ou plusieurs couches aval de prédiction d'au moins une classe, sous la forme d'un score, ledit deuxième réseau étant entraîné sur des sous-images d'entrainement comprenant des objets, chaque sous-image d'entrainement étant globalement annotée; et

F. pour l'image acquise :

a. le calcul d'un vecteur de caractéristique calculés pour les sous-images ;

b. l'application d'un modèle de prédiction d'au moins une classe pour les objets présents dans l'échantillon en fonction du vecteur de caractéristiques, ledit modèle de prédiction étant entrainé sur des vecteurs de caractéristiques calculés à partir d'images d'entrainement.

FIG. 1

EP 4 621 730 A1

FIG. 2

FIG. 3

FIG. 4

FIG. 5

66_CNN

Réseau de neurones convolutif

66

patchs avec
annotation forte

66_BDD

maxpool
maxpool
maxpool
maxpool
maxpool

Deph 64
3x3 conv
Conv1_1
Conv1_2

Deph 128
3x3 conv
Conv2_1
Conv2_2

Deph 256
3x3 conv
Conv3_1
Conv3_2
Conv3_3
Conv3_4

Deph 512
3x3 conv
Conv4_1
Conv4_2
Conv4_3
Conv4_4

Deph 512
3x3 conv
Conv5_1
Conv5_2
Conv5_3
Conv5_4

Extracteur de caractéristiques

*Emb*

FC1    FC2

softmax

*Class_image*

FIG. 6A

EP 4 621 730 A1

66_CNN

66    Réseau de neurones convolutif

maxpool
maxpool    maxpool    maxpool    flattening

Emb

Class_image

softmax

FC1  FC2

Deph 64
3x3 conv
Conv1_1
Conv1_2

Deph 128
3x3 conv
Conv2_1
Conv2_2

Deph 256
3x3 conv
Conv3_1
Conv3_2
Conv3_3
Conv3_4

Deph 512
3x3 conv
Conv4_1
Conv4_2
Conv4_3
Conv4_4

Deph 512
3x3 conv
Conv5_1
Conv5_2
Conv5_3
Conv5_4

Extracteur de caractéristiques

**FIG. 6B**

66_CNN

66    Réseau de neurones convolutif

maxpool
maxpool    maxpool    maxpool    flattening

Emb

Class_image

softmax

FC1

FC2

Deph 64
3x3 conv
Conv1_1
Conv1_2

Deph 128
3x3 conv
Conv2_1
Conv2_2

Deph 256
3x3 conv
Conv3_1
Conv3_2
Conv3_3
Conv3_4

Deph 512
3x3 conv
Conv4_1
Conv4_2
Conv4_3
Conv4_4

Deph 512
3x3 conv
Conv5_1
Conv5_2
Conv5_3
Conv5_4

Extracteur de caractéristiques

**FIG. 6C**

**FIG. 7**

64

Image

$Class\_scores(1,1)$

$Class\_scores(i,j)$

$Class\_scores(I,J)$

80

$$\max_{i,j} Class\_scores(i,j)(q)$$

$$X - centile_{i,j}(Class\_scores(i,j)(q))$$

82

Random forest

Dataset

Decision Tree-1    Decision Tree-2    Decision Tree-n

Result-1    Result-2    Result-N

Majority
Voting/Averaging

Final Result

Class_image

Conf_index

**FIG. 8**

Annotations / Predictions comparison
Image ID: Sample-17_Field-06_XY222_Species-Escherichia coli

Sous-image
Original image

Heat maps des différentes classes
prédites par le réseau 66  CNN

Annotations / Predictions comparison
Image ID: Sample-2_Field-16_XY221_Species-Enterococcus faecalis

Sous-image

**Fig. 9B**

Heat maps des différentes classes
prédites par le réseau 66_CNN

FIG. 9A

**FIG. 10B**

Accuracy / Rejection Rate

**FIG. 10A**

Constitution d'une base de données d'entraînement d'images, de sous-images et de patchs avec leurs classes — 102

Entraînement du CNN 66_CNN — 104

Constitution bases de données de vecteurs de caractéristiques par sous-image avec leurs classes — 106

Sous-image (1,1) $Emb_{1,1}(1,1)$
Sous-image (1,2) $Emb_{1,2}(1,1)$
Sous-image (N,M) $Emb_{N,M}(1,1)$ ... $Emb_{N,M}(K,L)$

Entraînement du prédicteur 68 — 108

Constitution bases de données de distributions de probabilité annotées par leurs classes — 110

Image 1 / Image 2 / Image E

Entraînement du Random Forest 82 — 112

Perf. OK?

Non

Oui — 114

Fin

100

Précision vs taux de rejet avec un zoom sur les valeurs de précision

Fig. 10B

**FIG. 11A**

| | Précision | | F1 macro | |
|---|---|---|---|---|
| | Cross-validation moyenne | Test | Cross-validation moyenne | Test |
| Patch | 0.86 | 0.82 | 0.49 | 0.52 |
| Sous Image | 0.88 | 0.90 | 0.72 | 0.75 |
| Lame | 0.92 | 0.89 | 0.83 | 0.80 |

Matrice de confusion de la cross-validation

Matrice de confusion de test

**FIG. 11B**

**FIG. 12A**

| | Précision | | F1 macro | |
|---|---|---|---|---|
| | Cross-validation moyenne | Test | Cross-validation moyenne | Test |
| Patch | 0.66 | 0.74 | 0.31 | 0.35 |
| Sous Image | 0.72 | 0.78 | 0.52 | 0.59 |
| Lame | 0.76 | 0.77 | 0.63 | 0.64 |

Matrice de confusion de la cross-validation

Matrice de confusion de test

**FIG. 12B**

FIG. 14

2000
2002
2004
2012
2006
2008
2010

FIG. 13

2000
2002
2004
2012
2006
2008
2010

**FIG. 15**

Europäisches
Patentamt

European
Patent Office

Office européen
des brevets

# RAPPORT DE RECHERCHE EUROPEENNE

Numéro de la demande

EP 24 16 5898

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | BIN LI ET AL: "Dual-stream Multiple Instance Learning Network for Whole Slide Image Classification with Self-supervised Contrastive Learning", ARXIV.ORG, CORNELL UNIVERSITY LIBRARY, 201 OLIN LIBRARY CORNELL UNIVERSITY ITHACA, NY 14853, 2 avril 2021 (2021-04-02), XP081927026, * le document en entier * | 1-27 | INV. G06V10/44 G06V10/50 G06V10/82 G06V20/69 C12Q1/00 |
| A | CA 3 133 826 A1 (TEMPUS LABS INC [US]) 1 octobre 2020 (2020-10-01) * le document en entier * | 1-27 | |
| A | US 2020/357516 A1 (KIRBY JAMES E [US] ET AL) 12 novembre 2020 (2020-11-12) * le document en entier * | 1-27 | |

DOMAINES TECHNIQUES RECHERCHES (IPC)

G06V
G01N
C12Q

Le présent rapport a été établi pour toutes les revendications

| Lieu de la recherche | Date d'achèvement de la recherche | Examinateur |
|---|---|---|
| La Haye | 13 août 2024 | de Bont, Emma |

CATEGORIE DES DOCUMENTS CITES

X : particulièrement pertinent à lui seul
Y : particulièrement pertinent en combinaison avec un
autre document de la même catégorie
A : arrière-plan technologique
O : divulgation non-écrite
P : document intercalaire

T : théorie ou principe à la base de l'invention
E : document de brevet antérieur, mais publié à la
date de dépôt ou après cette date
D : cité dans la demande
L : cité pour d'autres raisons

.............................................................................

& : membre de la même famille, document correspondant

EPO FORM 1503 03.82 (P04C02)

**EP 4 621 730 A1**

## ANNEXE AU RAPPORT DE RECHERCHE EUROPEENNE
## RELATIF A LA DEMANDE DE BREVET EUROPEEN NO.

EP 24 16 5898

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

13-08-2024

| Document brevet cité au rapport de recherche | Date de publication | Membre(s) de la famille de brevet(s) | Date de publication |
|---|---|---|---|
| CA 3133826 A1 | 01-10-2020 | AU 2020248416 A1 | 07-10-2021 |
| | | CA 3133826 A1 | 01-10-2020 |
| | | EP 3991171 A1 | 04-05-2022 |
| | | JP 2022527264 A | 01-06-2022 |
| | | KR 20210145778 A | 02-12-2021 |
| | | SG 11202109958S A | 28-10-2021 |
| | | WO 2020198380 A1 | 01-10-2020 |
| US 2020357516 A1 | 12-11-2020 | US 2020357516 A1 | 12-11-2020 |
| | | WO 2019104003 A1 | 31-05-2019 |

**RÉFÉRENCES CITÉES DANS LA DESCRIPTION**

**Documents brevets cités dans la description**

- EP 4307051 A
- EP 4172699 A
- WO 2016075279 A
- WO 2017077238 A
- WO 17207184 A

**Littérature non-brevet citée dans la description**

- **M. ILSE et al.** Attention-based Deep Multiple Instance Learning. *arXiv:1802.04712v4 [cs.LG*, 28 June 2018
- **BREIMAN et al.** Random Forests. *Machine Learning*, 2001, vol. 45 (1), 5-32
- **S. BERG et al.** ilastik: interactive machine learning for (bio)image analysis. *Nature Methods*, 2019